# EUROPEAN PATENT APPLICATION

(11) **EP 3 070 102 A1**
(43) Date of publication of application: **21.09.2016**
(21) Application number: 15159611.1
(22) Date of filing: 18.03.2015
(51) Int. Cl.: C07K 16/28, A61K 39/395, A61P 35/00, A61K 39/00

(54) **COMBINATION THERAPY OF ANTIBODIES HUMAN CD40 ACTIVATING ANTIBODIES AND ANTI HUMAN PLD-1 ANTIBODIES**

(71) Applicant: F. Hoffmann-La Roche AG, 4070 Basel (CH)
(72) Inventor: The designation of the inventor has not yet been filed
(74) Representative: Beyermann, Jochen Carl

(57) **Abstract**

The present disclosure relates to a pharmaceutical product for the treatment of a proliferative disease, comprising the combination of an antibody, or an antigen-binding portion thereof, that specifically binds to human CD40; and a PD-L1 antibody; and optionally a 3^{rd} component which comprises as an active ingredient a cytokine inhibitor.

## Description

The present invention relates to the combination therapy of specific antibodies which bind human CD40 with specific antibodies which bind human PD-L1. These combinations are particularly useful in the field of cancer therapy.

### BACKGROUND OF THE INVENTION

Immunomodulatory antibodies offer an treatment approach and might be used to directly potentiate anti-tumor immune responses or as adjuvants for anti-cancer vaccines (Melero, I., et al. Nat Rev Cancer 7, 2007, 95-106). Agonistic anti-CD40 antibodies constitute one of the most effective classes of these reagents. CD40 is a cell-surface member of the tumor necrosis factor superfamily expressed on antigen presenting cells (APCs) such as dendritic cells, B cells and macrophages. Preclinical studies with anti-CD40 agonists suggest that triggering CD40 with crosslinking antibodies on antigen presenting cells (APCs) can substitute for CD4 T cell help, normally provided via CD40 ligand, and facilitate the activation as well as expansion of CD8 effector T cells (Li, F. et al, Science 333, 2011, 1030-1034). In addition CD40-activated macrophages may also exert direct tumoricidal functions (Beatty, G. L., et al. Science 331, 2011, 1612-1616; Vonderheide, R. H., et al., Oncoimmunology 2, 2013, e23033). CD40 agonists are disclosed in WO 2003/040170.

Recently, it has been discovered that T cell dysfunction or anergy occurs concurrently with an induced and sustained expression of the inhibitory receptor, programmed death 1 polypeptide (PD-1). As a result, therapeutic targeting PD-1 and other molecules which signal through interactions with PD-1, such as programmed death ligand 1 (PD-L1) and programmed death ligand 2 (PD-L2) are an area of intense interest. The inhibition of PD-L1 signaling has been proposed as a means to enhance T cell immunity for the treatment of cancer (*e.g*., tumor immunity) and infection, including both acute and chronic (*e.g*., persistent) infection. Antibodies against PD-L1 are described e.g. in WO 2010/077634.

Co-stimulation or the provision of two distinct signals to T-cells is a widely accepted model of lymphocyte activation of resting T lymphocytes by antigen-presenting cells (APCs). Lafferty et al., Aust. J. Exp. Biol. Med. Sci. 53: 27-42 (1975). The mechanism of co-stimulation is of therapeutic interest because the manipulation of co-stimulatory signals has shown to provide a means to either enhance or terminate cell-based immune response. However, as an optimal therapy co-targeting CD40 and PD-L1 has yet to be commercialized, a significant unmet medical need exists.

### SUMMARY OF THE INVENTION

The invention comprises the combination therapy of an antibody which binds to human CD40 with an antibody which binds to human PD-L1 for use in the treatment of a proliferative disease, in particular cancer.

The invention further comprises a pharmaceutical product comprising A) a first component comprising as an active ingredient an antibody or an antigen-binding portion thereof that specifically binds to and activates human CD40; and B) a second component comprising as an active ingredient a PD-L1 antibody; for the combined, sequential or simultaneous, treatment of a proliferative disease, in particular cancer.

The invention further comprises the use of an antibody, or an antigen-binding portion thereof, that specifically binds to and activates human CD40; and a PD-L1 antibody for the manufacture of a medicament for the combined, sequential or simultaneous, treatment of a proliferative disease, such as cancer preferably solid tumors.

The invention further comprises a kit comprsing the pharmaceutical product as defined above.

The invention further comprises a method of treating a patient suffering from a proliferative disease, in particular cancer, said method comprises the combined, simultaneous or sequential administration of an anti-CD40 - and a PD-L1 antibody.

### BRIEF DESCRIPTION OF THE FIGURES

Fig. 1: Analysis of Combined muFGK4.5 (anti-CD40) and Anti-PD-L1 Tumor Growth Inhibition in the Orthotopic Panc02-H7-Fluc Pancreatic Carcinoma Model **(a)** versus vehicle and monotherapy control, **(b)** individual tumor growth curves of mice from the a-CD40 + a-PDL1 combination group.
**Fig. 2****:** Individual Mice treated with combined muFGK4.5 and Anti-PD-L1 on Day 22 of treatment.
**Fig. 3****:** Immune Pharmacodynamic (PD) Analysis of combined muFGK4.5 and anti PD-L1 antibody ("Combo") in the Orthotopic Panc02 H7-Fluc Pancreatic Carcinoma Model versus control and monotherapy.
**Fig. 4****:** Dosages and dosage schedules for clinical trials involving combination treatment with human anti CD40 - and anti PD-L1 antibodies. Dose of PD-L1 antibody is fixed at 1200mg.
**Fig. 5****:** Addition of anti-TNF alpha as 3^{rd} component prevents weight loss in mice induced by treatment with anti CD40/anti PD-L1 combination.
**Fig. 6****:** Addition of anti-TNF alpha as 3^{rd} component does not compromise anti-tumor activity of anti CD40/anti PD-L1 combination treatment.

### SEQUENCE LISTINGS

- SEQ ID NO 1:: human CD40
- SEQ ID NO 2:: human PD-L1
- SEQ ID NO 3:: light chain variable domain (VL) of a CD40 antibody according to the present invention.
- SEQ ID NO 4:: heavy chain variable domain (VH) of a CD40 antibody according to the present invention.
- SEQ ID NO: 5: heavy chain variable domain VH variant 1, anti-PD-L1 243.55
- SEQ ID NO: 6: heavy chain variable domain VH variant 2, anti-PD-L1 243.55
- SEQ ID NO: 7: heavy chain variable domain VH variant 3, anti-PD-L1 243.55
- SEQ ID NO: 8: light chain variable domain VL variant 1, anti-PD-L1 243.55
- SEQ ID NO: 9: light chain variable domain VL variant 2, anti-PD-L1 243.55
- SEQ ID NO: 10: light chain variable domain VL variant 3, anti-PD-L1 243.55
- SEQ ID NO: 11: light chain variable domain VL variant 4, anti-PD-L1 243.55
- SEQ ID NO: 12: light chain variable domain VL variant 5, anti-PD-L1 243.55
- SEQ ID NO: 13: light chain variable domain VL variant 6, anti-PD-L1 243.55
- SEQ ID NO: 14: light chain variable domain VL variant 7, anti-PD-L1 243.55
- SEQ ID NO: 15: light chain variable domain VL variant 8, anti-PD-L1 243.55
- SEQ ID NO: 16: light chain variable domain VL variant 9, anti-PD-L1 243.55
- SEQ ID NO: 17: light chain variable domain VL variant 10, anti-PD-L1 243.55
- SEQ ID NO: 18: light chain variable domain VL variant 11, anti-PD-L1 243.55
- SEQ ID NO: 19: light chain variable domain VL variant 12, anti-PD-L1 243.55
- SEQ ID NO: 20: light chain variable domain VL variant 13, anti-PD-L1 243.55
- SEQ ID NO: 21: light chain variable domain VL variant 14, anti-PD-L1 243.55
- SEQ ID NO: 22: light chain variable domain VL variant 15, anti-PD-L1 243.55
- SEQ ID NO: 23: light chain variable domain VL variant 16, anti-PD-L1 243.55

### DETAILED DESCRIPTION OF THE INVENTION

PD-L1 (programmed death ligand 1) is one of two ligands (PD-L1 and PD-L2) that bind PD-1, an inhibitory receptor expressed on T cells following T cell activation, which is sustained in states of chronic stimulation such as in chronic infection or cancer (see e.g. Blank C et al, Cancer Immunol Immunother, 2005, 54:307-14; and Keir ME et al, Annual Rev Immunol, 2008, 26:677-704). Aberrant expression of PD-L1 on tumor cells has been reported to make them much less immunogenic (Dong H et al, Nat Med, 2002, 8:793-800) and to impede anti tumor immunity, resulting in immune evasion (Blank C et al, Cancer Immunol Immunother, 2007, 56:739-45). Blockade of PD L1 or PD 1 with monoclonal antibodies results in strong and often rapid anti tumor effects in several mouse tumor models (see e.g. Iwai Y. et al, Proc Natl Acad Sci USA, 2002; 99:12293-7; Strome SE et al, Cancer Res 2003; 63:6501-5), suggesting that tumor specific T cells may be present in the tumor micro environment in an inactive or repressed state, and that inhibition of the PD 1/PD L1 pathway may reinvigorate tumor specific, T cell responses. Based on the experimental data, blockade of PD-L1 or PD-1 is expected to enhance the ongoing immune response against tumor antigens (Merelli B et al, Crit Rev Oncol Hematol., 2014; 89:140-65). Antibodies against PD-L1 are for example described e.g. in WO 2010/077634.

CD40, a member of the tumor necrosis factor receptor (TNFR) superfamily, is a critical regulator of the anti tumor immune response via its expression on antigen presenting cells (APCs) that include B lymphocytes, dendritic cells (DCs), and monocytes (see e.g. Grewal IS et al, Ann Rev Immunol, 1998;16:111-35; Van Kooten C et al, J Leukoc. Biol, 2000;67:2-17; or O'Sullivan B et al, Crit Rev Immunol. 2003;23(1 2):83-107). CD40 stimulated DCs up regulate antigen processing and presentation pathways and migrate to lymph nodes to activate naive T cells. Agonist CD40 antibodies were shown to substitute the function of CD4+ lymphocytes resulting in cytotoxic T lymphocyte (CTL) expansion able to clear established lymphoma in murine models (see e.g. Sotomayor EM et al, Nature Medicine, 1999;5(7):780-7; Gladue RP et al, Cancer Immunol Immunother, 2011;60(7):1009-17). CD40 agonists trigger immune stimulation by activating host APCs, which then drive T cell responses directed against the tumor (see e.g. Vonderheide RH, Clin Cancer Res, 2007;13:1083-8).

The combination of a CD40 agonist and a PD-L1 inhibitor thus may trigger priming and expansion of anti tumor T cells in the secondary lymphoid organs through CD40 agonistic activity, and remove PD 1 / PD-L1 mediated, anti tumor T cell immunosuppression, at the same time. However, immune therapy and, more specifically T cell mediated immune response, may also result in severe adverse events (AE's) such as infusion related reactions, cytokine-release (CRS, "Cytokine Storms" or "hypercytokinemia"), and immune related toxicities.

According to the present invention, it has now been found that the combined, sequential or simultaneous, application of a CD40 agonist and a PD-L1 inhibitor results in a more than additive (i.e. synergistic) inhibition of tumor growth and even in remission of individual tumors *in vivo.* In a preferred embodiment, the administration of the two components, i.e. CD40 agonist and PD-L1 inhibitor, is sequential.

Therefore, in one embodiment, the present invention provides a synergistic combination comprising a CD40 agonist and a PD-L1 inhibitor.

In another embodiment, the present invention provides dosages and dosage regimen for acceptable (i.e. tolerable) application of said CD40 agonist and said PD-L1 inhibitor in humans. These dosages and dosage regimen reduce or eliminate the risk of immune related toxicities and improve safety profile and tolerability of the present combination treatment.

The human CD40 antigen is a 50 kDa cell surface glycoprotein which belongs to the Tumor Necrosis Factor Receptor (TNF-R) family (Stamenkovic et al., EMBO J. 8:1403-10 (1989)). It is also known as "Tumor necrosis factor receptor superfamily member 5". Alternative designations include B-cell surface antigen 40, Bp50, CD40L receptor, CDw40, CDW40, MGC9013, p50 or TNFRSF5. It is for example registered under UniProt Entry No. P25942. In one embodiment human CD40 antigen has the sequence according to SEQ ID NO: 1 (see Table 1).

**Table 1: Protein sequence of human CD40 antigen**

| | |
|---|---|
| SEQ ID NO:1 | MVRLPLQCVLWGCLLTAVHPEPPTACREKQYLINSQC CSLCQPGQKLVSDCTEFTETECLPCGESEFLDTWNRE THCHQHKYCDPNLGLRVQQKGTSETDTICTCEEGWH CTSEACESCVLHRSCSPGFGVKQIATGVSDTICEPCPV GFFSNVSSAFEKCHPWTSCETKDLVVQQAGTNKTDV VCGPQDRLRALVVIPIIFGILFAILLVLVFIKKVAKKPT NKAPHPKQEPQEINFPDDLPGSNTAAPVQETLHGCQP VTQEDGKESR ISVQERQ |

The human PD-L1 (or PDL1) antigen is also designated as "Programmed cell death 1 ligand 1" or CD274 molecule. Alternative designations comprise B7-H, B7H1, B7-H1, B7 homolog 1, MGC142294, MGC142296, PDCD1L1, PDCD1LG1, PDCD1 ligand 1, PDL1, PD-L1, Programmed death ligand 1. In one embodiment the human PD-L1 antigen has the sequence of SEQ ID NO:2 (Table 2), as for example registered as UniProt Entry No. Q9NZQ7.

**Table 2: Protein sequence of human PD-L1 antigen**

| | |
|---|---|
| SEQ ID NO:2 | MRIFAVFIFMTYWHLLNAFTVTVPKDLYVVEYGSNM TIECKFPVEKQLDLAALIVYWEMEDKNIIQFVHGEEDL KVQHSSYRQRARLLKDQLSLGNAALQITDVKLQDAG VYRCMISYGGADYKRITVKVNAPYNKINQRILVVDPV TSEHELTCQAEGYPKAEVIWTSSDHQVLSGKTTTTNS |
| | KREEKLFNVTSTLRINTTTNEIFYCTFRRLDPEENHTAE LVIPELPLAHPPNERTHLVILGAILLCLGVALTFIFRLR KGRMMDVKKCGIQDTNSKKQSDTHLEET |

According to the present invention, an "anti-CD40 antibody" (or "CD40 antibody", "A-CD40 antibody") is an antibody binding, or specifically binding to and activating human CD40. In one embodiment human CD40 has the sequence according to SEQ ID NO: 1. As used herein, "binding to human CD40" or "specifically binding to human CD40" or "which binds to human CD40" or "anti-CD40 antibody" refers to an antibody specifically binding to the human CD40 antigen with a binding affinity of K_{D}-value of 1.0 x 10⁻⁸ mol/l or less, in one embodiment of a K_{D}-value of 1.0 x10⁻⁹ mol/l or less. The binding affinity is determined with a standard binding assay, such as surface plasmon resonance technique (BIAcore®, GE-Healthcare Uppsala, Sweden). Thus an "antibody binding to and activating human CD40" as used herein refers to an antibody specifically binding to the human CD40 antigen with a binding affinity of KD 1.0 x 10⁻⁸ mol/l or less (in one embodiment 1.0 x 10⁻⁸ mol/l - 1.0 x 10⁻¹³ mol/l), in on embodiment of a KD 1.0 x10⁻⁹ mol/l or less (in one embodiment 1.0 x 10⁻⁹ mol/l - 1.0 x 10⁻¹³ mol/l). In another embodiment, the anti-CD40 antibody according to the present invention binds to human CD40 with a K_{D} of 4 x 10⁻¹⁰ M or less.

In one embodiment of the present invention, the antibody which binds to human CD40 is an agonist ("CD40 agonist"). An "agonist" combines with a receptor on a cell and initiates a reaction or activity that is similar to or the same as that initiated by a natural ligand of the receptor. An "CD40 agonist" induces any or all of, but not limited to, the following responses: B cell proliferation and/or differentiation; upregulation of intercellular adhesion via such molecules as ICAM- 1, E-selectin, VC AM, and the like; secretion of pro-inflammatory cytokines such as IL-1, IL-6, IL-8, IL-12, TNF, and the like; signal transduction through the CD40 receptor by such pathways as TRAF {e.g., TRAF2 and/or TRAF3), MAP kinases such as NIK (NF-kB inducing kinase), I-kappa B kinases (IKK /.beta.), transcription factor NF-kB, Ras and the MEK/ERK pathway, the PI3K AKT pathway, the P38 MAPK pathway, and the like; transduction of an anti-apoptotic signal by such molecules as XIAP, mcl-1, bcl-x, and the like; B and/or T cell memory generation; B cell antibody production; B cell isotype switching, up-regulation of cell-surface expression of MHC Class II and CD80/86, and the like.

By agonist activity is intended an agonist activity of at least 30%, 10 35%, 40%, 45%, 50%, 60%, 70%, 75%, 80%, 85%, 90%, 95%, or 100% greater than the agonist activity induced by a negative control as measured in an assay of a B cell response.

In another embodiment an CD40 agonist has an agonist activity that is at least 2-fold greater or at least 3-fold greater than the agonist activity induced by a negative control as measured in an assay of a B cell response.

Thus, for example, where the B cell response of interest is B cell proliferation, agonist activity would be induction of a level of B cell proliferation that is at least 2-fold greater or at least 3-fold greater than the level of B cell proliferation induced by a negative control.

The "CD40 agonist" as used herein includes any moiety that agonizes the CD40/CD40L interaction. Typically these moieties will be agonistic CD40 antibodies or agonistic CD40L polypeptides. These antibodies include by way of example human antibodies, chimeric antibodies, humanized antibodies, bispecific antibodies, scFvs, and antibody fragments that specifically agonize the CD40/CD40L binding interaction. In one embodiment the agonistic CD40 antibody will comprise a chimeric, fully human or humanized CD40 antibody. In another preferred embodiment the agonistic CD40 antibody will comprise a chimeric, fully human or humanized CD40 antibody

In one embodiment, said CD40 antibody is a fully human antibody of the IgG2 subclass. In yet another embodiment said antibody is any of the anti-CD40 antibodies as specifically disclosed in WO2003/040170. In still another embodiment the CD40 agonist according to the present invention is selected from the group of antibodies designated 3.1.1, 7.1.2, 10.8.3, 15. 1.1, 21.4.1, 21.2.1, 22.1.1, 23.5.1, 23.25.1, 23.29.1 and 24.2.1 according to WO2003/040170. Hybridomas secreting those antibodies have been deposited in accordance with the Budapest Treaty. Deposit Numbers can be found in para [0250] of WO2003/040170. In another embodiment, the CD40 antibody according to the present invention comprises the heavy chain - and light chain variable domain amino acid sequences of antibody 21.4.1 (ATCC Deposit No. PTA-3605). In yet another embodiment, the CD40 antibody according to the present invention consists of the heavy chain - and light chain amino acid sequences of antibody 21.4.1 (ATCC Deposit No. PTA-3605).

In one embodiment, the human anti-CD40 antibody according to the present invention comprises a light chain variable domain of amino acid SEQ ID NO: 3 and a heavy chain variable domain of amino acid SEQ ID NO: 4 (Table 3).

**Table 3: Amino acid sequences of the light (VL) - and heavy (VH) chain variable domain of a CD40 agonist according to the present invention.**

| | |
|---|---|
| VL (Amino Acid Sequence) SEQ ID NO: 3 | DIQMTQSPSSVSASVGDRVTITCRASQGIYSWLAWYQQKP GKAPNLLIYTASTLQSGVPSRFSGSGSGTDFTLTISSLQPED FATYYCQQ ANIFPLTFGGGTKVEIK |
| VH (Amino Acid Sequence) SEQ ID NO: 4 | QVQLVQSGAEVKKPGASVKVSCKASGYTFTGYYMHWV RQAPGQGLEWMGWINPDSGGTNYAQKFQGRVTMTRDTS ISTAYMELNRLRSDDTAVYYCARDQPLGYCTNGVCSYFD YWGQGT LVTVSS |

According to the present invention, a "PD-L1 antibody" is an antibody binding to or specifically binding to human PD-L1. In one embodiment human PD-L1 has the sequence according to SEQ ID NO:2. As used herein, "binding to human PD-L1" or "specifically binding to human PD-L1" or "which binds to human PD-L1" or "anti- PD-L1 antibody" refers to an antibody specifically binding to the human PD-L1 antigen with a binding affinity of KD-value of 1.0 x 10⁻⁸ mol/l or lower, in one embodiment of a KD-value of 1.0 x 10⁻⁹ mol/l or lower. The binding affinity is determined with a standard binding assay, such as surface plasmon resonance technique (BIAcore®, GE-Healthcare Uppsala, Sweden). Thus an "antibody binding to human PD-L1" as used herein refers to an antibody specifically binding to the human PD-L1 antigen with a binding affinity of KD 1.0 x 10⁻⁸ mol/l or lower (in one embodiment 1.0 x 10⁻⁸ mol/l - 1.0 x 10⁻¹³ mol/l), in on embodiment of a KD 1.0 x10⁻⁹ mol/l or lower (in one embodiment 1.0 x 10⁻⁹ mol/l - 1.0 x 10⁻¹³ mol/l).

In one embodiment the antibody which binds to human PD-L1 used in the combination therapy described herein is selected from the group consisting of:
243.55.S70, 243.55.H1, 243.55.H12, 243.55.H37, 243.55.H70, 243.55.H89, 243.55.S1, 243.55.5, 243.55.8 , 243.55.30, 243.55.34 , 243.55.S37 , 243.55.49, 243.55.51, 243.55.62 , and 243.55.84.

These antibodies are described in WO 2010/77634 (sequences are shown in Figure 11 of WO 2010/77634) and are characterized in comprising the following heavy chain variable domain (VH) and light chain variable domain (VL) sequences (Table 4):

**Table 4: Combinations of VH and VL for selected PD-L1 antibodies**

| anti-PD-L1 antibody | amino acid sequence of the heavy chain variable domain VH, SEQ ID NO: | amino acid sequence of the light chain variable domain VL, SEQ ID NO: |
|---|---|---|
| 243.55.S70 | 5 | 8 |
| 243.55.H1 | 6 | 9 |
| 243.55.H12 | 6 | 10 |
| 243.55.H37 | 6 | 11 |
| 243.55.H70 | 6 | 12 |
| 243.55.H89 | 6 | 13 |
| 243.55.S1 | 6 | 14 |
| 243.55.5 | 6 | 15 |
| 243.55.8 | 6 | 16 |
| 243.55.30 | 6 | 17 |
| 243.55.34 | 6 | 18 |
| 243.55.S37 | 6 | 19 |
| 243.55.49 | 6 | 20 |
| 243.55.51 | 6 | 21 |
| 243.55.62 | 6 | 22 |
| 243.55.84 | 7 | 23 |

**Table 5: Sequences of SEQ ID NO's according to Table 4**

| SEQ ID NO: | |
|---|---|
| 5 | Glu Val Gln Leu Val Glu Ser Gly Gly Gly Leu Val Gln Pro Gly Gly Ser Leu Arg Leu Ser Cys Ala Ala Ser Gly Phe Thr Phe Ser Asp Ser Trp Ile His Trp Val Arg Gln Ala Pro Gly Lys Gly Leu Glu Trp Val Ala Trp Ile Ser Pro Tyr Gly Gly Ser Thr Tyr Tyr Ala Asp Ser Val Lys Gly Arg Phe Thr Ile Ser Ala Asp Thr Ser Lys Asn Thr Ala Tyr Leu Gln Met Asn Ser Leu Arg Ala Glu Asp Thr Ala Val Tyr Tyr Cys Ala Arg Arg |
| | His Trp Pro Gly Gly Phe Asp Tyr Trp Gly Gln Gly Thr Leu Val Thr Val Ser Ala |
| 6 | Glu Val Gln Leu Val Glu Ser Gly Gly Gly Leu Val Gln Pro Gly Gly Ser Leu Arg Leu Ser Cys Ala Ala Ser Gly Phe Thr Phe Ser Asp Ser Trp Ile His Trp Val Arg Gln Ala Pro Gly Lys Gly Leu Glu Trp Val Ala Trp Ile Ser Pro Tyr Gly Gly Ser Thr Tyr Tyr Ala Asp Ser Val Lys Gly Arg Phe Thr Ile Ser Ala Asp Thr Ser Lys Asn Thr Ala Tyr Leu Gln Met Asn Ser Leu Arg Ala Glu Asp Thr Ala Val Tyr Tyr Cys Ala Arg Arg His Trp Pro Gly Gly Phe Asp Tyr Trp Gly Gln Gly Thr Leu Val Thr Val Ser Ala |
| 7 | Glu Val Gln Leu Val Glu Ser Gly Gly Gly Leu Val Gln Pro Gly Gly Ser Leu Arg Leu Ser Cys Ala Ala Ser Gly Phe Thr Phe Ser Gly Ser Trp Ile His Trp Val Arg Gln Ala Pro Gly Lys Gly Leu Glu Trp Val Ala Trp Ile Leu Pro Tyr Gly Gly Ser Ser Tyr Tyr Ala Asp Ser Val Lys Gly Arg Phe Thr Ile Ser Ala Asp Thr Ser Lys Asn Thr Ala Tyr Leu Gln Met Asn Ser Leu Arg Ala Glu Asp Thr Ala Val Tyr Tyr Cys Ala Arg Arg His Trp Pro Gly Gly Phe Asp Tyr Trp Gly Gln Gly Thr Leu Val Thr Val Ser Ala |
| 8 | Asp Ile Gln Met Thr Gln Ser Pro Ser Ser Leu Ser Ala Ser Val Gly Asp Arg Val Thr Ile Thr Cys Arg Ala Ser Gln Asp Val Ser Thr Ala Val Ala Trp Tyr Gln Gln Lys Pro Gly Lys Ala Pro Lys Leu Leu Ile Tyr Ser Ala Ser Phe Leu Tyr Ser Gly Val Pro Ser Arg Phe Ser Gly Ser Gly Ser Gly Thr Asp Phe Thr Leu Thr Ile Ser Ser Leu Gln Pro Glu Asp Phe Ala Thr Tyr Tyr Cys Gln Gln Tyr Leu Tyr His Pro Ala Thr Phe Gly Gln Gly Thr Lys Val Glu Ile Lys Arg |
| 9 | Asp Ile Gln Met Thr Gln Ser Pro Ser Ser Leu Ser Ala Ser Val Gly Asp Arg Val Thr Ile Thr Cys Arg Ala Ser Gln Asp Val Ser Thr Ala Val Ala Trp Tyr Gln Gln |
| | Lys Pro Gly Lys Ala Pro Lys Leu Leu Ile Tyr Ser Ala Ser Phe Leu Tyr Ser Gly Val Pro Ser Arg Phe Ser Gly Ser Gly Ser Gly Thr Asp Phe Thr Leu Thr Ile Ser Ser Leu Gln Pro Glu Asp Phe Ala Thr Tyr Tyr Cys Gln Gln Tyr Tyr Asn Val Pro Trp Thr Phe Gly Gln Gly Thr Lys Val Glu Ile Lys Arg |
| 10 | Asp Ile Gln Met Thr Gln Ser Pro Ser Ser Leu Ser Ala Ser Val Gly Asp Arg Val Thr Ile Thr Cys Arg Ala Ser Gln Asp Val Ser Thr Ala Val Ala Trp Tyr Gln Gln Lys Pro Gly Lys Ala Pro Lys Leu Leu Ile Tyr Ser Ala Ser Phe Leu Tyr Ser Gly Val Pro Ser Arg Phe Ser Gly Ser Gly Ser Gly Thr Asp Phe Thr Leu Thr Ile Ser Ser Leu Gln Pro Glu Asp Phe Ala Thr Tyr Tyr Cys Gln Gln Tyr Tyr Ala Pro Pro Trp Thr Phe Gly Gln Gly Thr Lys Val Glu Ile Lys Arg |
| 11 | Asp Ile Gln Met Thr Gln Ser Pro Ser Ser Leu Ser Ala Ser Val Gly Asp Arg Val Thr Ile Thr Cys Arg Ala Ser Gln Asp Val Ser Thr Ala Val Ala Trp Tyr Gln Gln Lys Pro Gly Lys Ala Pro Lys Leu Leu Ile Tyr Ser Ala Ser Phe Leu Tyr Ser Gly Val Pro Ser Arg Phe Ser Gly Ser Gly Ser Gly Thr Asp Phe Thr Leu Thr Ile Ser Ser Leu Gln Pro Glu Asp Phe Ala Thr Tyr Tyr Cys Gln Gln Tyr Tyr Thr Val Pro Trp Thr Phe Gly Gln Gly Thr Lys Val Glu Ile Lys Arg |
| 12 | Asp Ile Gln Met Thr Gln Ser Pro Ser Ser Leu Ser Ala Ser Val Gly Asp Arg Val Thr Ile Thr Cys Arg Ala Ser Gln Val Ile Asn Thr Phe Leu Ala Trp Tyr Gln Gln Lys Pro Gly Lys Ala Pro Lys Leu Leu Ile Tyr Ser Ala Ser Thr Leu Ala Ser Gly Val Pro Ser Arg Phe Ser Gly Ser Gly Ser Gly Thr Asp Phe Thr Leu Thr Ile Ser Ser Leu Gln Pro Glu Asp Phe Ala Thr Tyr Tyr Cys Gln Gln Tyr Tyr Thr Val Pro Arg Thr Phe Gly Gln Gly Thr Lys Val Glu Ile Lys Arg |
| 13 | Asp Ile Gln Met Thr Gln Ser Pro Ser Ser Leu Ser Ala Ser Val Gly Asp Arg Val Thr Ile Thr Cys Arg Ala Ser Gln Asp Val Ser Thr Ala Val Ala Trp Tyr Gln Gln Lys Pro Gly Lys Ala Pro Lys Leu Leu Ile Tyr Ser Ala Ser Phe Leu Tyr Ser Gly Val Pro Ser Arg Phe Ser Gly Ser Gly Ser Gly Thr Asp Phe Thr Leu Thr Ile Ser Ser Leu Gln Pro Glu Asp Phe Ala Thr Tyr Tyr Cys Gln Gln Gly Tyr Gly Val Pro Arg Thr Phe Gly Gln Gly Thr Lys Val Glu Ile Lys Arg |
| 14 | Asp Ile Gln Met Thr Gln Ser Pro Ser Ser Leu Ser Ala Ser Val Gly Asp Arg Val Thr Ile Thr Cys Arg Ala Ser Gln Asp Val Ser Thr Ala Val Ala Trp Tyr Gln Gln Lys Pro Gly Lys Ala Pro Lys Leu Leu Ile Tyr Ser Ala Ser Phe Leu Tyr Ser Gly Val Pro Ser Arg Phe Ser Gly Ser Gly Ser Gly Thr Asp Phe Thr Leu Thr Ile Ser Ser Leu Gln Pro Glu Asp Phe Ala Thr Tyr Tyr Cys Gln Gln Tyr Leu Phe Thr Pro Pro Thr Phe Gly Gln Gly Thr Lys Val Glu Ile Lys Arg |
| 15 | Asp Ile Gln Met Thr Gln Ser Pro Ser Ser Leu Ser Ala Ser Val Gly Asp Arg Val Thr Ile Thr Cys Arg Ala Ser Gln Asp Val Ser Thr Ala Val Ala Trp Tyr Gln Gln Lys Pro Gly Lys Ala Pro Lys Leu Leu Ile Tyr Ser Ala Ser Phe Leu Tyr Ser Gly Val Pro Ser Arg Phe Ser Gly Ser Gly Ser Gly Thr Asp Phe Thr Leu Thr Ile Ser Ser Leu Gln Pro Glu Asp Phe Ala Thr Tyr Tyr Cys Gln Gln Tyr Phe Ile Thr Pro Thr Thr Phe Gly Gln Gly Thr Lys Val Glu Ile Lys Arg |
| 16 | Asp Ile Gln Met Thr Gln Ser Pro Ser Ser Leu Ser Ala Ser Val Gly Asp Arg Val Thr Ile Thr Cys Arg Ala Ser Gln Asp Val Ser Thr Ala Val Ala Trp Tyr Gln Gln Lys Pro Gly Lys Ala Pro Lys Leu Leu Ile Tyr Ser Ala Ser Phe Leu Tyr Ser Gly Val Pro Ser Arg Phe Ser Gly Ser Gly Ser Gly Thr Asp Phe Thr Leu Thr Ile Ser Ser Leu Gln Pro Glu Asp Phe Ala Thr Tyr Tyr Cys Gln Gln Tyr Tyr Tyr Thr Pro Pro Thr Phe Gly Gln Gly |
| | Thr Lys Val Glu Ile Lys Arg |
| 17 | Asp Ile Gln Met Thr Gln Ser Pro Ser Ser Leu Ser Ala Ser Val Gly Asp Arg Val Thr Ile Thr Cys Arg Ala Ser Gln Asp Val Ser Thr Ala Val Ala Trp Tyr Gln Gln Lys Pro Gly Lys Ala Pro Lys Leu Leu Ile Tyr Ser Ala Ser Phe Leu Tyr Ser Gly Val Pro Ser Arg Phe Ser Gly Ser Gly Ser Gly Thr Asp Phe Thr Leu Thr Ile Ser Ser Leu Gln Pro Glu Asp Phe Ala Thr Tyr Tyr Cys Gln Gln Phe Phe Tyr Thr Pro Pro Thr Phe Gly Gln Gly Thr Lys Val Glu Ile Lys Arg |
| 18 | Asp Ile Gln Met Thr Gln Ser Pro Ser Ser Leu Ser Ala Ser Val Gly Asp Arg Val Thr Ile Thr Cys Arg Ala Ser Gln Asp Val Ser Thr Ala Val Ala Trp Tyr Gln Gln Lys Pro Gly Lys Ala Pro Lys Leu Leu Ile Tyr Ser Ala Ser Phe Leu Tyr Ser Gly Val Pro Ser Arg Phe Ser Gly Ser Gly Ser Gly Thr Asp Phe Thr Leu Thr Ile Ser Ser Leu Gln Pro Glu Asp Phe Ala Thr Tyr Tyr Cys Gln Gln Ser Leu Phe Thr Pro Pro Thr Phe Gly Gln Gly Thr Lys Val Glu Ile Lys Arg |
| 19 | Asp Ile Gln Met Thr Gln Ser Pro Ser Ser Leu Ser Ala Ser Val Gly Asp Arg Val Thr Ile Thr Cys Arg Ala Ser Gln Asp Val Ser Thr Ala Val Ala Trp Tyr Gln Gln Lys Pro Gly Lys Ala Pro Lys Leu Leu Ile Tyr Ser Ala Ser Phe Leu Tyr Ser Gly Val Pro Ser Arg Phe Ser Gly Ser Gly Ser Gly Thr Asp Phe Thr Leu Thr Ile Ser Ser Leu Gln Pro Glu Asp Phe Ala Thr Tyr Tyr Cys Gln Gln Ser Leu Tyr Thr Pro Pro Thr Phe Gly Gln Gly Thr Lys Val Glu Ile Lys Arg |
| 20 | Asp Ile Gln Met Thr Gln Ser Pro Ser Ser Leu Ser Ala Ser Val Gly Asp Arg Val Thr Ile Thr Cys Arg Ala Ser Gln Asp Val Ser Thr Ala Val Ala Trp Tyr Gln Gln Lys Pro Gly Lys Ala Pro Lys Leu Leu Ile Tyr Ser Ala Ser Phe Leu Tyr Ser Gly Val Pro Ser Arg Phe Ser Gly Ser Gly Ser Gly Thr Asp Phe Thr Leu Thr Ile Ser Ser |
| | Leu Gln Pro Glu Asp Phe Ala Thr Tyr Tyr Cys Gln Gln Ser Trp Tyr His Pro Pro Thr Phe Gly Gln Gly Thr Lys Val Glu Ile Lys Arg |
| 21 | Asp Ile Gln Met Thr Gln Ser Pro Ser Ser Leu Ser Ala Ser Val Gly Asp Arg Val Thr Ile Thr Cys Arg Ala Ser Gln Asp Val Ser Thr Ala Val Ala Trp Tyr Gln Gln Lys Pro Gly Lys Ala Pro Lys Leu Leu Ile Tyr Ser Ala Ser Phe Leu Tyr Ser Gly Val Pro Ser Arg Phe Ser Gly Ser Gly Ser Gly Thr Asp Phe Thr Leu Thr Ile Ser Ser Leu Gln Pro Glu Asp Phe Ala Thr Tyr Tyr Cys Gln Gln Tyr Phe Tyr Ile Pro Pro Thr Phe Gly Gln Gly Thr Lys Val Glu Ile Lys Arg |
| 22 | Asp Ile Gln Met Thr Gln Ser Pro Ser Ser Leu Ser Ala Ser Val Gly Asp Arg Val Thr Ile Thr Cys Arg Ala Ser Gln Asp Val Ser Thr Ala Val Ala Trp Tyr Gln Gln Lys Pro Gly Lys Ala Pro Lys Leu Leu Ile Tyr Ser Ala Ser Phe Leu Tyr Ser Gly Val Pro Ser Arg Phe Ser Gly Ser Gly Ser Gly Thr Asp Phe Thr Leu Thr Ile Ser Ser Leu Gln Pro Glu Asp Phe Ala Thr Tyr Tyr Cys Gln Gln Tyr Trp Tyr Thr Pro Thr Thr Phe Gly Gln Gly Thr Lys Val Glu Ile Lys Arg |
| 23 | Asp Ile Gln Met Thr Gln Ser Pro Ser Ser Leu Ser Ala Ser Val Gly Asp Arg Val Thr Ile Thr Cys Arg Ala Ser Gln Asp Val Ser Thr Ala Val Ala Trp Tyr Gln Gln Lys Pro Gly Lys Ala Pro Lys Leu Leu Ile Tyr Ser Ala Ser Phe Leu Tyr Ser Gly Val Pro Ser Arg Phe Ser Ser Gly Ser Gly Thr Asp Phe Thr Leu Thr Ile Ser Ser Leu Gln Pro Glu Asp Phe Ala Thr Tyr Tyr Cys Gln Gln Ser Tyr Phe Ile Pro Pro Thr Phe Gly Gln Gly Thr Lys Val Glu Ile Lys Arg |

Therefore, in accordance with the present invention, the antibody which binds to human PD-L1 (i.e. the antibody of component B) is characterized by
a) a heavy chain variable domain VH of SEQ ID NO:5 and a light chain variable domain VL of SEQ ID NO:8, or
b) a heavy chain variable domain VH of SEQ ID NO:6 and a light chain variable domain VL of SEQ ID NO:9, or
c) a heavy chain variable domain VH of SEQ ID NO:6 and a light chain variable domain VL of SEQ ID NO: 10, or
d) a heavy chain variable domain VH of SEQ ID NO:6 and a light chain variable domain VL of SEQ ID NO: 11, or
e) a heavy chain variable domain VH of SEQ ID NO:6 and a light chain variable domain VL of SEQ ID NO: 12, or
f) a heavy chain variable domain VH of SEQ ID NO:6 and a light chain variable domain VL of SEQ ID NO: 13, or
g) a heavy chain variable domain VH of SEQ ID NO:6 and a light chain variable domain VL of SEQ ID NO: 14, or
h) a heavy chain variable domain VH of SEQ ID NO:6 and a light chain variable domain VL of SEQ ID NO: 15, or
i) a heavy chain variable domain VH of SEQ ID NO:6 and a light chain variable domain VL of SEQ ID NO:16, or
j) a heavy chain variable domain VH of SEQ ID NO:6 and a light chain variable domain VL of SEQ ID NO: 17, or
k) a heavy chain variable domain VH of SEQ ID NO:6 and a light chain variable domain VL of SEQ ID NO:18, or
l) a heavy chain variable domain VH of SEQ ID NO:6 and a light chain variable domain VL of SEQ ID NO: 19, or
m) a heavy chain variable domain VH of SEQ ID NO:6 and a light chain variable domain VL of SEQ ID NO:20, or
n) a heavy chain variable domain VH of SEQ ID NO:6 and a light chain variable domain VL of SEQ ID NO:21, or
o) a heavy chain variable domain VH of SEQ ID NO:6 and a light chain variable domain VL of SEQ ID NO:22, or
p) a heavy chain variable domain VH of SEQ ID NO:7 and a light chain variable domain VL of SEQ ID NO:23.

Therefore, in one embodiment, the present invention provides a pharmaceutical product comprising A) a first component comprising as an active ingredient an antibody or an antigen-binding portion thereof that specifically binds to and activates human CD40; and B) a second component comprising as an active ingredient a PD-L1 antibody; for the combined, sequential or simultaneous, treatment of a proliferative disease, in particular cancer.

Within this embodiment, the antibody in A) is a fully human antibody of the IgG2 subclass which binds to human CD40 with a K_{D} of 4 x 10⁻¹⁰ M or less; or
an antibody comprising a VL of SEQ ID NO:3 and a VH of SEQ ID NO:4; or
an antibody comprising the heavy chain - and light chain variable domain amino acid sequences of antibody 21.4.1 (ATCC Deposit No. PTA-3605); or
an antibody comprising the heavy chain - and light chain amino acid sequences of antibody 21.4.1 (ATCC Deposit No. PTA-3605); and
the antibody in B) is any of the PD-L1 antibodies mentioned in a) to p) herein before.

In yet another embodiment, the present invention provides a pharmaceutical product comprising A) a first component comprising as an active ingredient a fully human antibody of the IgG2 subclass which binds to human CD40 with a K_{D} of 4 x 10⁻¹⁰ M or less; and B) a second component comprising as an active ingredient a PD-L1 antibody selected from an antibody comprising
a) a heavy chain variable domain VH of SEQ ID NO:5 and a light chain variable domain VL of SEQ ID NO:8, or
b) a heavy chain variable domain VH of SEQ ID NO:6 and a light chain variable domain VL of SEQ ID NO:9, or
c) a heavy chain variable domain VH of SEQ ID NO:6 and a light chain variable domain VL of SEQ ID NO: 10, or
d) a heavy chain variable domain VH of SEQ ID NO:6 and a light chain variable domain VL of SEQ ID NO: 11, or
e) a heavy chain variable domain VH of SEQ ID NO:6 and a light chain variable domain VL of SEQ ID NO: 12, or
f) a heavy chain variable domain VH of SEQ ID NO:6 and a light chain variable domain VL of SEQ ID NO: 13, or
g) a heavy chain variable domain VH of SEQ ID NO:6 and a light chain variable domain VL of SEQ ID NO:14, or
h) a heavy chain variable domain VH of SEQ ID NO:6 and a light chain variable domain VL of SEQ ID NO: 15, or
i) a heavy chain variable domain VH of SEQ ID NO:6 and a light chain variable domain VL of SEQ ID NO:16, or
j) a heavy chain variable domain VH of SEQ ID NO:6 and a light chain variable domain VL of SEQ ID NO: 17, or
k) a heavy chain variable domain VH of SEQ ID NO:6 and a light chain variable domain VL of SEQ ID NO:18, or
l) a heavy chain variable domain VH of SEQ ID NO:6 and a light chain variable domain VL of SEQ ID NO:19, or
m) a heavy chain variable domain VH of SEQ ID NO:6 and a light chain variable domain VL of SEQ ID NO:20, or
n) a heavy chain variable domain VH of SEQ ID NO:6 and a light chain variable domain VL of SEQ ID NO:21, or
o) a heavy chain variable domain VH of SEQ ID NO:6 and a light chain variable domain VL of SEQ ID NO:22, or
p) a heavy chain variable domain VH of SEQ ID NO:7 and a light chain variable domain VL of SEQ ID NO:23; for the combined, sequential or simultaneous, treatment of a proliferative disease, in particular cancer.

In yet another embodiment, the present invention provides a pharmaceutical product comprising A) a first component comprising as an active ingredient an antibody comprising a VL of SEQ ID NO:3 and a VH of SEQ ID NO:4; and B) a second component comprising as an active ingredient a PD-L1 antibody selected from an antibody comprising
a) a heavy chain variable domain VH of SEQ ID NO:5 and a light chain variable domain VL of SEQ ID NO:8, or
b) a heavy chain variable domain VH of SEQ ID NO:6 and a light chain variable domain VL of SEQ ID NO:9, or
c) a heavy chain variable domain VH of SEQ ID NO:6 and a light chain variable domain VL of SEQ ID NO: 10, or
d) a heavy chain variable domain VH of SEQ ID NO:6 and a light chain variable domain VL of SEQ ID NO: 11, or
e) a heavy chain variable domain VH of SEQ ID NO:6 and a light chain variable domain VL of SEQ ID NO: 12, or
f) a heavy chain variable domain VH of SEQ ID NO:6 and a light chain variable domain VL of SEQ ID NO: 13, or
g) a heavy chain variable domain VH of SEQ ID NO:6 and a light chain variable domain VL of SEQ ID NO:14, or
h) a heavy chain variable domain VH of SEQ ID NO:6 and a light chain variable domain VL of SEQ ID NO: 15, or
i) a heavy chain variable domain VH of SEQ ID NO:6 and a light chain variable domain VL of SEQ ID NO:16, or
j) a heavy chain variable domain VH of SEQ ID NO:6 and a light chain variable domain VL of SEQ ID NO: 17, or
k) a heavy chain variable domain VH of SEQ ID NO:6 and a light chain variable domain VL of SEQ ID NO:18, or
l) a heavy chain variable domain VH of SEQ ID NO:6 and a light chain variable domain VL of SEQ ID NO:19, or
m) a heavy chain variable domain VH of SEQ ID NO:6 and a light chain variable domain VL of SEQ ID NO:20, or
n) a heavy chain variable domain VH of SEQ ID NO:6 and a light chain variable domain VL of SEQ ID NO:21, or
o) a heavy chain variable domain VH of SEQ ID NO:6 and a light chain variable domain VL of SEQ ID NO:22, or
p) a heavy chain variable domain VH of SEQ ID NO:7 and a light chain variable domain VL of SEQ ID NO:23;
for the combined, sequential or simultaneous, treatment of a proliferative disease, in particular cancer.

In yet another embodiment, the present invention provides a pharmaceutical product comprising A) a first component comprising as an active ingredient an antibody comprising the heavy chain - and light chain variable domain amino acid sequences of antibody 21.4.1 (ATCC Deposit No. PTA-3605); and B) a second component comprising as an active ingredient a PD-L1 antibody selected from an antibody comprising
a) a heavy chain variable domain VH of SEQ ID NO:5 and a light chain variable domain VL of SEQ ID NO:8, or
b) a heavy chain variable domain VH of SEQ ID NO:6 and a light chain variable domain VL of SEQ ID NO:9, or
c) a heavy chain variable domain VH of SEQ ID NO:6 and a light chain variable domain VL of SEQ ID NO: 10, or
d) a heavy chain variable domain VH of SEQ ID NO:6 and a light chain variable domain VL of SEQ ID NO: 11, or
e) a heavy chain variable domain VH of SEQ ID NO:6 and a light chain variable domain VL of SEQ ID NO: 12, or
f) a heavy chain variable domain VH of SEQ ID NO:6 and a light chain variable domain VL of SEQ ID NO: 13, or
g) a heavy chain variable domain VH of SEQ ID NO:6 and a light chain variable domain VL of SEQ ID NO:14, or
h) a heavy chain variable domain VH of SEQ ID NO:6 and a light chain variable domain VL of SEQ ID NO: 15, or
i) a heavy chain variable domain VH of SEQ ID NO:6 and a light chain variable domain VL of SEQ ID NO:16, or
j) a heavy chain variable domain VH of SEQ ID NO:6 and a light chain variable domain VL of SEQ ID NO: 17, or
k) a heavy chain variable domain VH of SEQ ID NO:6 and a light chain variable domain VL of SEQ ID NO:18, or
l) a heavy chain variable domain VH of SEQ ID NO:6 and a light chain variable domain VL of SEQ ID NO:19, or
m) a heavy chain variable domain VH of SEQ ID NO:6 and a light chain variable domain VL of SEQ ID NO:20, or
n) a heavy chain variable domain VH of SEQ ID NO:6 and a light chain variable domain VL of SEQ ID NO:21, or
o) a heavy chain variable domain VH of SEQ ID NO:6 and a light chain variable domain VL of SEQ ID NO:22, or
p) a heavy chain variable domain VH of SEQ ID NO:7 and a light chain variable domain VL of SEQ ID NO:23;
for the combined, sequential or simultaneous, treatment of a proliferative disease, in particular cancer.

In yet another embodiment, the present invention provides a pharmaceutical product comprising A) a first component comprising as an active ingredient the antibody comprising the heavy chain - and light chain amino acid sequences of antibody 21.4.1 (ATCC Deposit No. PTA-3605); and B) a second component comprising as an active ingredient a PD-L1 antibody selected from an antibody comprising
a) a heavy chain variable domain VH of SEQ ID NO:5 and a light chain variable domain VL of SEQ ID NO:8, or
b) a heavy chain variable domain VH of SEQ ID NO:6 and a light chain variable domain VL of SEQ ID NO:9, or
c) a heavy chain variable domain VH of SEQ ID NO:6 and a light chain variable domain VL of SEQ ID NO: 10, or
d) a heavy chain variable domain VH of SEQ ID NO:6 and a light chain variable domain VL of SEQ ID NO: 11, or
e) a heavy chain variable domain VH of SEQ ID NO:6 and a light chain variable domain VL of SEQ ID NO: 12, or
f) a heavy chain variable domain VH of SEQ ID NO:6 and a light chain variable domain VL of SEQ ID NO: 13, or
g) a heavy chain variable domain VH of SEQ ID NO:6 and a light chain variable domain VL of SEQ ID NO:14, or
h) a heavy chain variable domain VH of SEQ ID NO:6 and a light chain variable domain VL of SEQ ID NO:15, or
i) a heavy chain variable domain VH of SEQ ID NO:6 and a light chain variable domain VL of SEQ ID NO:16, or
j) a heavy chain variable domain VH of SEQ ID NO:6 and a light chain variable domain VL of SEQ ID NO: 17, or
k) a heavy chain variable domain VH of SEQ ID NO:6 and a light chain variable domain VL of SEQ ID NO:18, or
l) a heavy chain variable domain VH of SEQ ID NO:6 and a light chain variable domain VL of SEQ ID NO:19, or
m) a heavy chain variable domain VH of SEQ ID NO:6 and a light chain variable domain VL of SEQ ID NO:20, or
n) a heavy chain variable domain VH of SEQ ID NO:6 and a light chain variable domain VL of SEQ ID NO:21, or
o) a heavy chain variable domain VH of SEQ ID NO:6 and a light chain variable domain VL of SEQ ID NO:22, or
p) a heavy chain variable domain VH of SEQ ID NO:7 and a light chain variable domain VL of SEQ ID NO:23;
for the combined, sequential or simultaneous, treatment of a proliferative disease, in particular cancer.

In yet another embodiment, the present invention provides a pharmaceutical product comprising A) a first component comprising as an active ingredient an antibody comprising a VL of SEQ ID NO:3 and a VH of SEQ ID NO:4; and B) a second component comprising as an active ingredient an antibody comprising a VL of SEQ ID NO:8 and a VH of SEQ ID NO:5; for the combined, sequential or simultaneous, treatment of a proliferative disease, in particular cancer.

In yet another embodiment, the present invention provides a pharmaceutical product comprising A) a first component comprising as an active ingredient an antibody comprising the heavy chain - and light chain variable domain amino acid sequences of antibody 21.4.1 (ATCC Deposit No. PTA-3605); and B) a second component comprising as an active ingredient an antibody comprising the heavy chain - and light chain variable domain amino acid sequences of antibody 243.55.S70; for the combined, sequential or simultaneous, treatment of a proliferative disease, in particular cancer.

In yet another embodiment, the present invention provides a pharmaceutical product comprising A) a first component comprising as an active ingredient an antibody comprising the heavy chain - and light chain amino acid sequences of antibody 21.4.1 (ATCC Deposit No. PTA-3605); and B) a second component comprising as an active ingredient an antibody comprising the heavy chain - and light chain amino acid sequences of antibody 243.55.S70; for the combined, sequential or simultaneous, treatment of a proliferative disease, in particular cancer.

In one embodiment, components A) and B) are administered separately, preferably with a time difference of 1, or 2, or 3, or 4, or 5, or 6, or 7 days. In another embodiment, the difference between administration of component A) and B) can be any time between 1 day and 21 days, preferably between 1 day and 14 days, or 1 day and 10 days, or 1 day and 7 days.

In another embodiment the present invention provides the use of an antibody, or an antigen-binding portion thereof, that specifically binds to and activates human CD40; and a PD-L1 antibody for the manufacture of a medicament for the combined, sequential or simultaneous, treatment of a proliferative disease, such as cancer preferably solid tumors.

The invention further comprises a method for the treatment of a patient in need of therapy, characterized by administering to the patient a therapeutically effective amount of an antibody according to the invention. The invention comprises the use of an antibody according to the invention for the described therapy.

Thus one embodiment of the invention are the CD40 antibodies, or antigen-binding portions thereof, described herein for use in the treatment of cancer in combination with an anti-PD-L1 antibody as described herein. The term "cancer" as used herein may be, for example, lung cancer, non small cell lung (NSCL) cancer, bronchioloalviolar cell lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, gastric cancer, colon cancer, breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, cancer of the bladder, cancer of the kidney or ureter, renal cell carcinoma, carcinoma of the renal pelvis, mesothelioma, hepatocellular cancer, biliary cancer, neoplasms of the central nervous system (CNS), spinal axis tumors, brain stem glioma, glioblastoma multiforme, astrocytomas, schwanomas, ependymonas, medulloblastomas, meningiomas, squamous cell carcinomas, pituitary adenoma, lymphoma, lymphocytic leukemia, including refractory versions of any of the above cancers, or a combination of one or more of the above cancers. In one preferred embodiment such cancer is a breast cancer, colorectal cancer, melanoma, head and neck cancer, lung cancer or prostate cancer. In one embodiment such cancer is a solid tumor selected from breast cancer, lung cancer, colon cancer, ovarian cancer, melanoma cancer, bladder cancer, renal cancer, kidney cancer, liver cancer, head and neck cancer, colorectal cancer, pancreatic cancer, gastric carcinoma cancer, esophageal cancer, mesothelioma or prostate cancer. In another embodiment such cacer is a hematological tumor such as for example, leukemia (such as AML, CLL), lymphoma, myelomas. In still another embodiment the cancer is breast cancer, lung cancer, colon cancer, colorectal cancer, pancreatic cancer, gastric cancer or prostate cancer.

In one embodiment the present combination therapy is for use in the prevention or treatment of metastasis.

In one embodiment the present combination therapy is for use in treating or delaying progression of an immune related disease such as tumor immunity.

In one embodiment the present combination therapy is for use in stimulating an immune response or function, such as T cell activity.

The term "epitope" denotes a protein determinant of human CD40 or PD-L1 capable of specifically binding to an antibody. Epitopes usually consist of chemically active surface groupings of molecules such as amino acids or sugar side chains and usually epitopes have specific three dimensional structural characteristics, as well as specific charge characteristics. Conformational and nonconformational epitopes are distinguished in that the binding to the former but not the latter is lost in the presence of denaturing solvents.

The "variable domain" (light chain variable domain VL, heavy chain variable domain VH) as used herein denotes each of the pair of light and heavy chain domains which are involved directly in binding the antibody to the antigen. The variable light and heavy chain domains have the same general structure and each domain comprises four framework (FR) regions whose sequences are widely conserved, connected by three "hypervariable regions" (or complementary determining regions, CDRs). The framework regions adopt a beta-sheet conformation and the CDRs may form loops connecting the beta-sheet structure. The CDRs in each chain are held in their three-dimensional structure by the framework regions and form together with the CDRs from the other chain the antigen binding site. The antibody's heavy and light chain CDR3 regions play a particularly important role in the binding specificity/affinity of the antibodies according to the invention and therefore provide a further object of the invention.

The term "antigen-binding portion of an antibody" when used herein refer to the amino acid residues of an antibody which are responsible for antigen-binding. The antigen-binding portion of an antibody comprises amino acid residues from the "complementary determining regions" or "CDRs". "Framework" or "FR" regions are those variable domain regions other than the hypervariable region residues as herein defined. Therefore, the light and heavy chain variable domains of an antibody comprise from N- to C-terminus the domains FR1, CDR1, FR2, CDR2, FR3, CDR3, and FR4. Especially, CDR3 of the heavy chain is the region which contributes most to antigen binding and defines the antibody's properties. CDR and FR regions are determined according to the standard definition of Kabat et al., Sequences of Proteins of Immunological Interest, 5th ed., Public Health Service, National Institutes of Health, Bethesda, MD (1991) and/or those residues from a "hypervariable loop".

In one embodiment of the present invention, the "antigen-binding portion" of an antibody that specifically binds to and activates human CD40 comprises CDR1, CDR2 and CDR3 of the heavy- and light chain variable domains of antibody 21.4.1 (ATCC Deposit No. PTA-3605).

The terms "nucleic acid" or "nucleic acid molecule", as used herein, are intended to include DNA molecules and RNA molecules. A nucleic acid molecule may be single-stranded or double-stranded, but preferably is double-stranded DNA.

The term "amino acid" as used within this application denotes the group of naturally occurring carboxy alpha-amino acids comprising alanine (three letter code: ala, one letter code: A), arginine (arg, R), asparagine (asn, N), aspartic acid (asp, D), cysteine (cys, C), glutamine (gln, Q), glutamic acid (glu, E), glycine (gly, G), histidine (his, H), isoleucine (ile, I), leucine (leu, L), lysine (lys, K), methionine (met, M), phenylalanine (phe, F), proline (pro, P), serine (ser, S), threonine (thr, T), tryptophan (trp, W), tyrosine (tyr, Y), and valine (val, V).

The "Fc part" of an antibody is not involved directly in binding of an antibody to an antigen, but exhibit various effector functions. A "Fc part of an antibody" is a term well known to the skilled artisan and defined on the basis of papain cleavage of antibodies. Depending on the amino acid sequence of the constant region of their heavy chains, antibodies or immunoglobulins are divided in the classes: IgA, IgD, IgE, IgG and IgM, and several of these may be further divided into subclasses (isotypes), e.g. IgG1, IgG2, IgG3, and IgG4, IgA1, and IgA2. According to the heavy chain constant regions the different classes of immunoglobulins are called α, δ, ε, γ, and µ, respectively. The Fc part of an antibody is directly involved in ADCC (antibody-dependent cell-mediated cytotoxicity) and CDC (complement-dependent cytotoxicity) based on complement activation, C1q binding and Fc receptor binding. Complement activation (CDC) is initiated by binding of complement factor C1q to the Fc part of most IgG antibody subclasses. While the influence of an antibody on the complement system is dependent on certain conditions, binding to C1q is caused by defined binding sites in the Fc part. Such binding sites are known in the state of the art and described e.g. by Boackle, R.J., et al., Nature 282 (1979) 742-743; Lukas, T.J., et al., J. Immunol. 127 (1981) 2555-2560; Brunhouse, R., and Cebra, J.J., Mol. Immunol. 16 (1979) 907-917; Burton, D.R., et al., Nature 288 (1980) 338-344; Thommesen, J.E., et al., Mol. Immunol. 37 (2000) 995-1004; Idusogie, E.E., et al., J. Immunol. 164 (2000) 4178-4184; Hezareh, M., et al., J. Virology 75 (2001) 12161-12168; Morgan, A., et al., Immunology 86 (1995) 319-324; EP 0 307 434. Such binding sites are e.g. L234, L235, D270, N297, E318, K320, K322, P331 and P329 (numbering according to EU index of Kabat, E.A., see below). Antibodies of subclass IgG1, IgG2 and IgG3 usually show complement activation and C1q and C3 binding, whereas IgG4 do not activate the complement system and do not bind C1q and C3.

In one embodiment the antibody according to the invention may comprise an Fc part derived from human origin and preferably all other parts of the human constant regions. As used herein the term "Fc part derived from human origin" denotes a Fc part which is either a Fc part of a human antibody of the subclass IgG1, or IgG2, or IgG3 or IgG4, preferably a Fc part from human IgG1 or IgG2 subclass, a mutated Fc part from human IgG1 or IgG2 subclass (in one embodiment with a mutation on L234A + L235A), a Fc part from human IgG4 subclass or a mutated Fc part from human IgG4 subclass, in one more specific embodiment with a mutation on S228P. In another embodiment said antibodies may have reduced or minimal effector function. In one embodiment the minimal effector function may result from an effectorless Fc mutation. In one embodiment the effectorless Fc mutation is L234A/L235A or L234A/L235A/P329G or N297A or D265A/N297A. In one embodiment the effectorless Fc mutation may be selected for each of the antibodies independently of each other from the group comprising L234A/L235A, L234A/L235A/P329G, N297A and D265A/N297A.

In one embodiment the antibodies according to the present invention are monoclonal antibodies. In another embodiment the antibodies according to the present invention are of human IgG class (i.e. of IgG1, or IgG2, or IgG3 or IgG4 subclass). In yet another embodiment, one antibody is preferably of the IgG2 subclass and the other is of the IgG1 or IgG4 subclass.

In one embodiment the antibody described herein is characterized in that the constant chains are of human origin. Such constant chains are well known in the state of the art and e.g. described by Kabat, E.A., (see e.g. Johnson, G. and Wu, T.T., Nucleic Acids Res. 28 (2000) 214-218).

The antibodies described herein are preferably produced by recombinant means. Such methods are widely known in the state of the art and comprise protein expression in prokaryotic and eukaryotic cells with subsequent isolation of the antibody polypeptide and usually purification to a pharmaceutically acceptable purity. For the protein expression nucleic acids encoding light and heavy chains or fragments thereof are inserted into expression vectors by standard methods. Expression is performed in appropriate prokaryotic or eukaryotic host cells, such as CHO cells, NS0 cells, SP2/0 cells, HEK293 cells, COS cells, yeast, or E. coli cells, and the antibody is recovered from the cells (from the supernatant or after cells lysis).

Recombinant production of antibodies is well-known in the state of the art and described, for example, in the review articles of Makrides, S.C., Protein Expr. Purif. 17 (1999) 183-202; Geisse, S., et al., Protein Expr. Purif. 8 (1996) 271-282; Kaufman, R.J., Mol. Biotechnol. 16 (2000) 151-161; Werner, R.G., Drug Res. 48 (1998) 870-880.

The antibodies may be present in whole cells, in a cell lysate, or in a partially purified, or substantially pure form. Purification is performed in order to eliminate other cellular components or other contaminants, e.g. other cellular nucleic acids or proteins, by standard techniques, including alkaline/SDS treatment, CsCl banding, column chromatography, agarose gel electrophoresis, and others well known in the art. See Ausubel, F., et al., ed. Current Protocols in Molecular Biology, Greene Publishing and Wiley Interscience, New York (1987).

Expression in NS0 cells is described by, e.g., Barnes, L.M., et al., Cytotechnology 32 (2000) 109-123; Barnes, L.M., et al., Biotech. Bioeng. 73 (2001) 261-270. Transient expression is described by, e.g., Durocher, Y., et al., Nucl. Acids. Res. 30 (2002) E9. Cloning of variable domains is described by Orlandi, R., et al., Proc. Natl. Acad. Sci. USA 86 (1989) 3833-3837; Carter, P., et al., Proc. Natl. Acad. Sci. USA 89 (1992) 4285-4289; Norderhaug, L., et al., J. Immunol. Methods 204 (1997) 77-87. A preferred transient expression system (HEK 293) is described by Schlaeger, E.-J. and Christensen, K., in Cytotechnology 30 (1999) 71-83, and by Schlaeger, E.-J., in J. Immunol. Methods 194 (1996) 191-199.

The heavy and light chain variable domains according to the invention are combined with sequences of promoter, translation initiation, constant region, 3' untranslated region, polyadenylation, and transcription termination to form expression vector constructs. The heavy and light chain expression constructs can be combined into a single vector, co-transfected, serially transfected, or separately transfected into host cells which are then fused to form a single host cell expressing both chains.

The control sequences that are suitable for prokaryotes, for example, include a promoter, optionally an operator sequence, and a ribosome binding site. Eukaryotic cells are known to utilize promoters, enhancers and polyadenylation signals.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading frame. However, enhancers do not have to be contiguous. Linking is accomplished by ligation at convenient restriction sites. If such sites do not exist, the synthetic oligonucleotide adaptors or linkers are used in accordance with conventional practice.

The monoclonal antibodies are suitably separated from the culture medium by conventional immunoglobulin purification procedures such as, for example, protein A-Sepharose, hydroxylapatite chromatography, gel electrophoresis, dialysis, or affinity chromatography. DNA and RNA encoding the monoclonal antibodies are readily isolated and sequenced using conventional procedures. The hybridoma cells can serve as a source of such DNA and RNA. Once isolated, the DNA may be inserted into expression vectors, which are then transfected into host cells such as HEK 293 cells, CHO cells, or myeloma cells that do not otherwise produce immunoglobulin protein, to obtain the synthesis of recombinant monoclonal antibodies in the host cells.

As used herein, the expressions "cell", "cell line", and "cell culture" are used interchangeably and all such designations include progeny. Thus, the words "transformants" and "transformed cells" include the primary subject cell and cultures derived therefrom without regard for the number of transfers. It is also understood that all progeny may not be precisely identical in DNA content, due to deliberate or inadvertent mutations. Variant progeny that have the same function or biological activity as screened for in the originally transformed cell are included.

Methods for producing the specific antibodies used in accordance with the present invention are also disclosed in WO2003/040170 (for anti-CD40 antibodies) and WO2010/77634 (for PD-L1 antibodies).

In another aspect, the present invention provides a composition, e.g. a pharmaceutical composition, containing one or a combination of monoclonal antibodies, or the antigen-binding portion thereof, of the present invention, formulated together with a pharmaceutically acceptable carrier.

As used herein, "pharmaceutically acceptable carrier" includes any and all solvents, dispersion media, coatings, antibacterial and antifungal agents, isotonic and absorption/resorption delaying agents, and the like that are physiologically compatible. Preferably, the carrier is suitable for injection or infusion.

A composition of the present invention can be administered by a variety of methods known in the art. As will be appreciated by the skilled artisan, the route and/or mode of administration will vary depending upon the desired results.

Pharmaceutically acceptable carriers include sterile aqueous solutions or dispersions and sterile powders for the preparation of sterile injectable solutions or dispersion. The use of such media and agents for pharmaceutically active substances is known in the art. In addition to water, the carrier can be, for example, an isotonic buffered saline solution.

Regardless of the route of administration selected, the compounds of the present invention, which may be used in a suitable hydrated form, and/or the pharmaceutical compositions of the present invention, are formulated into pharmaceutically acceptable dosage forms by conventional methods known to those of skill in the art.

Pharmaceutical compositions of the specific antibodies used in accordance with the present invention may be standard preparations known to the skilled person, and are for example disclosed in WO2003/040170 (for anti-CD40 antibodies) and WO2010/77634 (for PD-L1 antibodies).

Actual dosage levels of the active ingredients in the pharmaceutical compositions of the present invention may be varied so as to obtain an amount of the active ingredient which is effective to achieve the desired therapeutic response for a particular patient, composition, and mode of administration, without being toxic to the patient (effective amount). The selected dosage level will depend upon a variety of pharmacokinetic factors including the activity of the particular compositions of the present invention employed, or the ester, salt or amide thereof, the route of administration, the time of administration, the rate of excretion of the particular compound being employed, other drugs, compounds and/or materials used in combination with the particular compositions employed, the age, sex, weight, condition, general health and prior medical history of the patient being treated, and like factors well known in the medical arts.

The term "a method of treating" or its equivalent, when applied to, for example, cancer refers to a procedure or course of action that is designed to reduce or eliminate the number of cancer cells in a patient, or to alleviate the symptoms of a cancer. "A method of treating" cancer or another proliferative disorder does not necessarily mean that the cancer cells or other disorder will, in fact, be eliminated, that the number of cells or disorder will, in fact, be reduced, or that the symptoms of a cancer or other disorder will, in fact, be alleviated. Often, a method of treating cancer will be performed even with a low likelihood of success, but which, given the medical history and estimated survival expectancy of a patient, is nevertheless deemed to induce an overall beneficial course of action.

The terms "administered in combination with" or "co-administration", "coadministering", "combination therapy" or "combination treatment" refer to the administration of the anti-CD40 - and the anti-PD-L1 antibody as described herein e.g. as separate formulations/applications (or as one single formulation/application). The co-administration can be simultaneous or sequential in either order, wherein preferably there is a time period while both (or all) active agents simultaneously exert their biological activities. In one embodiment, either of the components A and B as used herein can be administered by its own, separated pharmaceutical preparation using an intravenous route (i.v.), e.g. through a continuous infusion, or a subcutaneous route (s.c). In one embodiment components A and B are co-administerd separately by an i.v. route/preparation. In another embodiment components A and B are co-administerd separately by an s.c. route/preparation. In still another embodiment components A and B are co-administerd separately one by an s.c. route/preparation and the other by an i.v. route/preparation.

It is self-evident that the antibodies are administered to the patient in a "therapeutically effective amount" (or simply "effective amount") which is the amount of the respective compound or combination that will elicit the biological or medical response of a tissue, system, animal or human that is being sought by the researcher, veterinarian, medical doctor or other clinician.

The dosages of each component in co-administration and the timing of co-administration will depend on the type (species, gender, age, weight, etc.) and condition of the patient being treated and the severity of the disease or condition being treated. In particular, treatments based on activation of the immune system generally may bear the risk of an overwhelming immune response, sometimes referred to as the Cytokine Release Syndrom (CRS), or Anti-Drug Antibody (ADA) responses. Finding a therapeutic, clinically relevant treatment regimen, i.e. tolerable and efficacious dose(s) and dosage schedule(s), involving CD40 agonists constitutes a currently unmet medical need.

Thus, in one embodiment, the components (A and B) are co-administered sequentially with each single component's dose being administered either on the same day in two separate administrations (i.v. or s.c.) or one of the components is administered on day 1 and the second is co-administered on any day between day 2 and day 42, or between day 2 and day 21, or between day 2 and 14, or between day 2 and day 7. In another embodiment, the second component is co-administered on day 2, or 7, or 14, or 21 after administration of the first component. In another embodiment, the second component is co-administered on day 2 or day 21 after administration of the first component.

In another embodiment, the first administered component is component A, i.e. an anti CD40 antibody as defined herein. Within this embodiment, component A is administered at a fixed dose between 4mg and 16mg, preferably at 4mg, or 8mg, or 16mg.

In another embodiment, component A is administered once at a dose of 16mg on day 1, followed by a dosing of component B on day 42 and further maintenance dosing of component B every 3 weeks, or every 14 or 7 days.

In another embodiment, component A is administered once at a dose of 16mg on day 1, followed by a first dosing of component B on day 21 and further maintenance dosing of component B every 3 weeks, or every 14 or 7 days.

In another embodiment, component A is administered once at a dose of 4mg on day 1, followed by a first dosing of component B on day 21 and further maintenance dosing of component B every 3 weeks, or every 14 or 7 days.

In another embodiment, component A is administered once at a dose of 4mg on day 1, followed by a first dosing of component B on day 14 and further maintenance dosing of component B every 3 weeks, or every 14 or 7 days.

In another embodiment, component A is administered once at a dose of 4mg on day 1, followed by a first dosing of component B on day 7 and further maintenance dosing of component B every 3 weeks, or every 14 or 7 days.

In another embodiment, component A is administered once at a dose of 16mg on day 1, followed by a first dosing of component B on day 14 and further maintenance dosing of component B every 3 weeks, or every 14 or 7 days.

In another embodiment, component A is administered once at a dose of 16mg on day 1, followed by a first dosing of component B on day 7 and further maintenance dosing of component B every 3 weeks, or every 14 or 7 days.

In another embodiment, component B is administered on day 1, followed by a single administration of component A on day 2 at a fixed dose between 4mg and 16mg; and further followed by maintenance dosing of component B every 3 weeks, or every 14 or 7 days. Within this embodiment, the fixed dose for component A is preferably selected from 4mg, 8mg, 14mg, 15mg or 16mg.

In yet another embodiment, component B is administered on day 1 of the treatment followed by further administration every 3 weeks (21 days), and component A is administered on day 2 of the treatment followed by 3 additional administrations each within a distance of 6 weeks (42days). Within this embodiment, component A is administered at a fixed dose between 4mg and 16mg, preferably at 4mg, 8mg, 14mg, 15mg or 16mg.

In yet another embodiment, component B is administered on day 1 of the treatment followed by further administration every 3 weeks (21 days), and component A is administered on day 2 of the treatment followed by 3 additional administrations each within a distance of 3 weeks (21 days). Within this embodiment, component A is administered at a fixed dose between 4mg and 16mg, preferably at 4mg, 8mg, 14mg, 15mg or 16mg.

Within all of the above embodiments in relation to administration/dosage schemes, component B (i.e. the anti PD-L1 antibody) is administered at a fixed dose of 1200mg. Treatment, or "maintenance dosing", with component B is continued until disease progression.

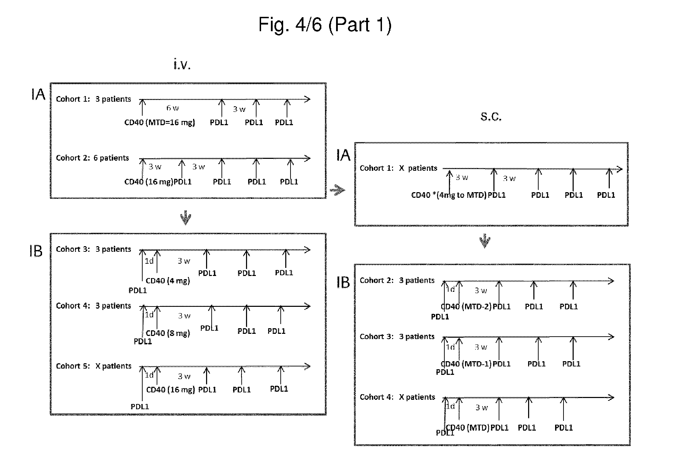

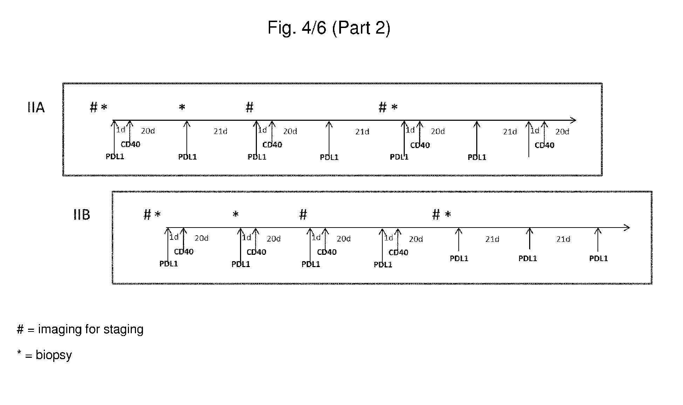

In yet another embodiment, the present invention provides any of the dosage schedules for combination therapy involving an anti-CD40 antibody and an anti-PD-L1 antibody, as shown in **Fig. 4****.**

Thus, in one embodiment, the present invention provides a pharmaceutical product comprising components A and B as defined herein before, wherein components A) and B) are administered separately.

In another embodiment, the present invention provides said pharmaceutical product, wherein component A and/or B is administered intravenously (i.v.) or subcutaneously (s.c.).

In yet another embodiment, the present invention provides said pharmaceutical product, wherein component A is administered at fixed doses between 4 and 16mg, and component B is administered at a fixed dose of 1200mg.

In still another embodiment, the present invention provides said pharmaceutical product, wherein administration of components A and B is separated by 1 to 42 days, preferably by 1, or 7, or 14 or 21 or 42 days.

In still another embodiment, the present invention provides said pharmaceutical product, wherein component A is administered from 1 to 4 times together with component B, and subsequently treatment continues only with component B until disease progression.

In another, more specific embodiment, the present invention provides any of the dosages schemes above, wherein component A is a component comprising as an active ingredient an antibody comprising a VL of SEQ ID NO:3 and a VH of SEQ ID NO:4; and component B is a component comprising as an active ingredient an antibody comprising a VL of SEQ ID NO:8 and a VH of SEQ ID NO:5.

In another embodiment the present invention provides the use of an antibody, or an antigen-binding portion thereof, that specifically binds to and activates human CD40; and a PD-L1 antibody for the manufacture of a medicament for the combined, sequential or simultaneous, treatment of a proliferative disease, such as cancer preferably solid tumors.

In addition to the anti-CD40 antibody in combination with the anti-PD-L1 antibody additional treatment options, such as chemotherapeutic agent or radiotherapy can be envisaged.

In one embodiment such additional chemotherapeutic agents, which may be administered with anti-CD40 antibody as described herein and the anti-PD-L1 antibody as described herein , include, but are not limited to, anti-neoplastic agents including alkylating agents including: nitrogen mustards, such as mechlorethamine, cyclophosphamide, ifosfamide, melphalan and chlorambucil; nitrosoureas, such as carmustine (BCNU), lomustine (CCNU), and semustine (methyl-CCNU); Temodal(TM) (temozolamide), ethylenimines/methylmelamine such as thriethylenemelamine (TEM), triethylene, thiophosphoramide (thiotepa), hexamethylmelamine (HMM, altretamine); alkyl sulfonates such as busulfan; triazines such as dacarbazine (DTIC); antimetabolites including folic acid analogs such as methotrexate and trimetrexate, pyrimidine analogs such as 5-fluorouracil (5FU), fluorodeoxyuridine, gemcitabine, cytosine arabinoside (AraC, cytarabine), 5-azacytidine, 2,2'-difluorodeoxycytidine, purine analogs such as 6-merca.rho.topurine, 6-thioguamne, azathioprine, T-deoxycoformycin (pentostatin), erythrohydroxynonyladenine (EHNA), fludarabine phosphate, and 2-chlorodeoxyadenosine (cladribine, 2-CdA); natural products including antimitotic drugs such as paclitaxel, vinca alkaloids including vinblastine (VLB), vincristine, and vinorelbine, taxotere, estramustine, and estramustine phosphate; pipodophylotoxins such as etoposide and teniposide; antibiotics such as actinomycin D, daunomycin (rubidomycin), doxorubicin, mitoxantrone, idarubicin, bleomycins, plicamycin (mithramycin), mitomycin C, and actinomycin; enzymes such as L-asparaginase; biological response modifiers such as interferon-alpha, IL-2, G-CSF and GM-CSF; miscellaneous agents including platinum coordination complexes such as oxaliplatin, cisplatin and carboplatin, anthracenediones such as mitoxantrone, substituted urea such as hydroxyurea, methylhydrazine derivatives including N- methylhydrazine (MIH) and procarbazine, adrenocortical suppressants such as mitotane (o, p-DDD) and aminoglutethimide; hormones and antagonists including adrenocorticosteroid antagonists such as prednisone and equivalents, dexamethasone and aminoglutethimide; Gemzar(TM) (gemcitabine), progestin such as hydroxyprogesterone caproate, medroxyprogesterone acetate and megestrol acetate; estrogen such as diethylstilbestrol and ethinyl estradiol equivalents; antiestrogen such as tamoxifen; androgens including testosterone propionate and fluoxymesterone/equivalents; antiandrogens such as flutamide, gonadotropin-releasing hormone analogs and leuprolide; and non-steroidal antiandrogens such as flutamide. Therapies targeting epigenetic mechanism including, but not limited to, histone deacetylase inhibitors, demethylating agents (e.g., Vidaza) and release of transcriptional repression (ATRA) therapies can also be combined with the antigen binding proteins. In one embodiment the chemotherapeutic agent is selected from the group consisting of taxanes (like e.g. paclitaxel (Taxol), docetaxel (Taxotere), modified paclitaxel (e.g., Abraxane and Opaxio), doxorubicin, sunitinib (Sutent), sorafenib (Nexavar), and other multikinase inhibitors, oxaliplatin, cisplatin and carboplatin, etoposide, gemcitabine, and vinblastine. In one embodiment the chemotherapeutic agent is selected from the group consisting of taxanes (like e.g. taxol (paclitaxel), docetaxel (Taxotere), modified paclitaxel (e.g. Abraxane and Opaxio). In one embodiment, the additional chemotherapeutic agent is selected from 5-fluorouracil (5-FU), leucovorin, irinotecan, or oxaliplatin. In one embodiment the chemotherapeutic agent is 5-fluorouracil, leucovorin and irinotecan (FOLFIRI). In one embodiment the chemotherapeutic agent is 5-fluorouracil, and oxaliplatin (FOLFOX).

Specific examples of combination therapies with additional chemotherapeutic agents include, for instance, therapies taxanes (e.g., docetaxel or paclitaxel) or a modified paclitaxel (e.g., Abraxane or Opaxio), doxorubicin), capecitabine and/or bevacizumab (Avastin) for the treatment of breast cancer; therapies with carboplatin, oxaliplatin, cisplatin, paclitaxel, doxorubicin (or modified doxorubicin (Caelyx or Doxil)), or topotecan (Hycamtin) for ovarian cancer, the therapies with a multi-kinase inhibitor, MKI, (Sutent, Nexavar, or 706) and/or doxorubicin for treatment of kidney cancer; therapies with oxaliplatin, cisplatin and/or radiation for the treatment of squamous cell carcinoma; therapies with taxol and/or carboplatin for the treatment of lung cancer.

The invention further provides a method for the manufacture of a medicament comprising an effective amount of an anti-CD40 antibody and/or a PD-L1 antibody according to the invention together with a pharmaceutically acceptable carrier.

The invention further provides a kit comprising a pharmaceutical product comprising A) an antibody, or an antigen-binding portion thereof, that specifically binds to and activates human CD40; and B) a PD-L1 antibody together with instructions for a skilled person, e.g. a physician, oncologist or other medical practitioner, how to use said pharmaceutical product as a medicament for the combined, sequential or simultaneous, treatment of a proliferative disease, such as cancer preferably solid tumors.

In still another embodiment, the pharmaceutical product according to the present invention, i.e. the product comprising components A and B as disclosed herein, can be administered together with a third component (C) wherein said third component is an anti-cytokine (cytokine inhibitor), such as for example anti-IL6 (a-IL6) and/or anti-TNF alpha (a-TNF α, anti-TNF α). Addition of said cytokine inhibitor has been found to improve tolerability while maintaining efficacy of the treatment with components A and B as disclosed above (see Example 2).

Therefore, in another embodiment, there is provided a pharmaceutical product comprising A) a first component comprising as an active ingredient an antibody or an antigen-binding portion thereof that specifically binds to and activates human CD40; and B) a second component comprising as an active ingredient a PD-L1 antibody; and C) a third component comprising as an active ingredient a cytokine inhibitor; for the combined, sequential or simultaneous, treatment of a proliferative disease, in particular cancer.

Within this embodiment, said cytokine inhibitor is a molecule which inhibits TNF α. A number of TNF α inhibitors, small molecules and antibodies, is approved for therapy in humans. Information about their applicable pharmaceutical preparations, routes of administration and doses are thus well known to the person of skill in the art. In one particular embodiment according to the present invention, the TNF α inhibitor is an antibody, preferably the human analogue of the antibody designated TN3-19.12. Other TNF alpha inhibitors which may be used in accordance with the present invention comprise Remicade® (infliximab), Enbrel® (etanercept), Humira® (adalimumab), Cimzia® (certolizumab pegol) and Simponi® (golimumab). Also, within this embodiment, component C can be administered simultaneously or separately from components A and B. Any of the dosage regimen disclosed herein for the administration of components A and B is applicable also for the triple combination. Component C is preferably administered simultaneously with component A and/or B, either in the same or different pharmaceutical preparation. Any dosage regimen as disclosed herein for components A and B together with component C forms another embodiment according to the present invention. Within this embodiment, component C is always administered when component A or B is also administered.

In another embodiment, administration of component C starts prior to administration of components A and B. Preferably, administration of component C starts on day 1 of the treatment, and components A and B are given from day 2 on, according to the dosage regimen defined for components A and B herein before.

In another embodiment, component C is preferably administered on days 1, 2, 3 and 4 of the treatment; component A is administered on day 2; and component B is administered on day 2 followed by weekly administration of component B alone or in combination with component C.

In yet another embodiment, component C is preferably administered on days 1, 2, 3 and 4 of the treatment; component A is administered on day 2; and component B is administered on day 9 followed by weekly administration of component B alone or in combination with component C.

In yet another embodiment, component C is preferably administered on days 1, 2, 3 and 4 of the treatment; component A is administered on day 2; and component B is administered on day 16 followed by weekly administration of component B alone or in combination with component C.

The following examples are provided to aid the understanding of the present invention, the true scope of which is set forth in the appended claims. It is understood that modifications can be made in the procedures set forth without departing from the spirit of the invention.

### EXAMPLES

### Example 1

This Example demonstrates the therapeutic efficacy of anti-murine CD40 in combination with anti-PDL-1 in an orthotopic Panc02-Fluc pancreatic syngeneic cancer model. In this study tumor growth will be evaluated via bioluminescence imaging two times a week.

### Experimental Schedule:

| **Study day** | **Experimental procedure** |
|---|---|
| 0 | Harvest Panc02-Fluc , prepare injection |
| 0 | Inject Panc02 intra-pancreatic |
| 2 | Imaging day 2 |
| 7 | 1^{st} Ab preparation |
| 7 | 1^{st} Ab injection |
| 7 | Imaging 7 |
| 8 | 24h bleeding |
| 9 | Imaging 9 |
| 10 | Scouts day 10 for Immuno-PD |
| 11 | Imaging 11 |
| 14 | 2nd Ab preparation |
| 14 | 2nd Ab injection |
| 15 | Imaging day 15 |
| 18 | Imaging day 18 |
| 21 | 3rd Ab preparation |
| 21 | 3rd Ab injection |
| 22 | Imaging day 22 |
| 25 | Imaging day 25 |
| 25 on | Histology |
| 25 on | Monitoring/postmortem |

### Study group

| **Group** | **No of animals** | **Panc02-Fluc injection 0.2 x10⁵** | **Compound** | **Dose (µg)** | **Route of administration** | **No of treatments** |
|---|---|---|---|---|---|---|
| 1 | 15 | yes | Vehicle | - | | 3 |
| 2 | 15 | yes | Anti-mouse CD40 + Anti-PDL1 | 10 mg/kg +10 mg/kg | Ab i.v. | 1 + 2 only PD-L1 |
| 3 | 15 | yes | Anti-PDL1 | 10 mg/kg | Ab i.v. | 3 |
| 4 | 15 | yes | Anti-mouse CD40 | 10 mg/kg | Ab i.v. | 1 |

The animals of each group will be sacrificed when the termination criteria has been met. Two scouts animal from vehicle group will be used to assess tumor burden at therapy initiation.

### Materials and Methods

### Cell culture and application

Panc-02-Fluce cells clone (human pancreatic carcinoma cells) were originally obtained from ATCC (American Type Culture Collection) and after expansion deposited in the Roche-Glycart internal cell bank. Panc-02-Fluc cells were cultured in RPMI medium containing 10% FCS (Sigma) and 1% of Glutamax + 500 ug/ml hygromycin..The cells were cultured at 37°C in a water-saturated atmosphere at 5 % CO2. A small incision was made in the left flank of the abdomen of anesthetized C57BL/6 mice. The peritoneal wall was opened and the pancreas carefully isolated with forceps. Ten microliters (0.2x106 cells in RPMI medium) of cell suspension were injected into the tail of the pancreas. Peritoneal wall and skin wounds were closed using 5/0 resorvable suture.

### Animals

Sixty Black 6 female mice; age 8-9 weeks at start of experiment (purchased from Charles Rivers, Germany) were maintained under specific-pathogen-free condition with daily cycles of 12 h light /12 h darkness according to committed guidelines (GV-Solas; Felasa; TierschG). Experimental study protocol was reviewed and approved by local government (P 2011-128). After arrival animals were maintained for one week to get accustomed to new environment and for observation. Continuous health monitoring was carried out on regular basis.

### Treatment

Mice were injected intra-pancreatic on study day 0 with 2x10⁵ of Panc02-Fluc cells in RPMI (passage 18 at a viability of 95.9 %). At day 7 mice were injected i.v., Vehicle (15 animals), 10 mg/kg a-CD40 (15animals), 10 mg/kg a-PD-L1, 10 mg/kg a-CD40 + 10 mg/kg a-PD-L1. The schedule was once a week for 3 weeks for a-PD-L1 and vehicle. A-CD40 antibody was only administered once at day 7.

### Test compounds (antibodies)

### Specification of compounds

| Compound | Lot.-Nr. | Formulation buffer | Conc. (mg/mL) |
|---|---|---|---|
| muIgG1 CD40 FGK4.5 B6 (Box AD,4) | sf W(3a) | 20 mM Histidine, 140 mM NaCl, 0.01% Tween-20, pH6.0 | 2.1 |
| muIgG1 GNE aPD-L1 DAPG (Box X,13) | sf W(2a) | 20 mM Histidine, 140 mM NaCl, pH6.0 | 2.29 |

The antibodies were stored at 4 degrees centigrade.

All mice were injected i.v. with 200 µL of the appropriate solution. The mice in the vehicle group were injected with Histidine buffer and the treatment group with the antibody. To obtain the proper amount of antibody, the antibody solutions were diluted with Histidine buffer when necessary. The maximum number of treatments for a mouse was three times.

### Antibodies preparation:

### 10 mg/kg equals 200 µg/mouse:

Below is calculated the volumes needed of the constructs per mouse:

| Antibody | Batch | Concentration (mg/mL) | Volume Protein (µL) | Volume Histidine buffer (µL) | Total Volume (µL) |
|---|---|---|---|---|---|
| muIgG1 CD40 FGK4.5 B6 (Box AD,4) | sf W(3a) | 2.1 | 95.2 | 104.8 | 200 |
| muIgG1 GNE aPD-L1 DAPG (Box X,13) | sf W(2a) | 2.29 | 87.3 | 112.7 | 200 |

### Investigations

### Monitoring

Animals were controlled daily for clinical symptoms and detection of adverse effects. Termination criteria for animals were clinical sickness, impaired locomotion, scruffy fur.

### Serum extraction for tumor marker analysis

Serum samples from all treated animals were collected three days before tumor cell injection, 24h after the first antibody treatment and at day of sacrifice. Samples were stored at -20° C.

### Autopsy

Mice were sacrificed according to the termination criteria. From all animals spleen and liver tumors were harvested for subsequent histopathological analysis (PFA, frozen).

### Sample processing

The pancreas tumours were resected and fixed immediately in formalin solution and subsequently processed for paraffin embedding (Leica Automatic Tissue Processor TP1020, Germany) and microtone 4µm sectioning (Leica RM2235 Rotary Microtome, Germany). Haematoxylin and Eosin staining was performed using standard protocol. Murine immune cells were detected using rat anti-mouse CD68 (AbD Serotec, Switzerland), in accordance with the manufacturer's instructions.

### Biochemical Analysts

### Rat IgG- ELISA and mEGFR IgG ELISA

Mice were bled 24h (study day 8) after the first antibody treatment and sera were analysed for the human-IgG concentration.Therapeutic antibody concentrations in serum were tested using an ELISA for Quantification of monoclonal antibody against CD20 (Roche Pharma Penzberg; TR-TNA5, Germany) according to the protocol. The reagents mAb<hfcy>IgG-Bi (M-R10Z8E9; Ch.02 GG); mAb<CD20>rH-IgG (RO5072759); mAb<hfcy>IgG-Dig (XOSU-Sux)(M-R10Z8E9;Ch.03); were kindly provided by J. Schleypen, Roche Penzberg. Serum samples were analysed in serial dilutions (1:2000; 1:20000; 1:200000). Absorption was measured using a measuring wavelength of 405 nm and a reference wavelength of 492 nm (VersaMax tunable microplate reader, Molecular Devices).

### Results

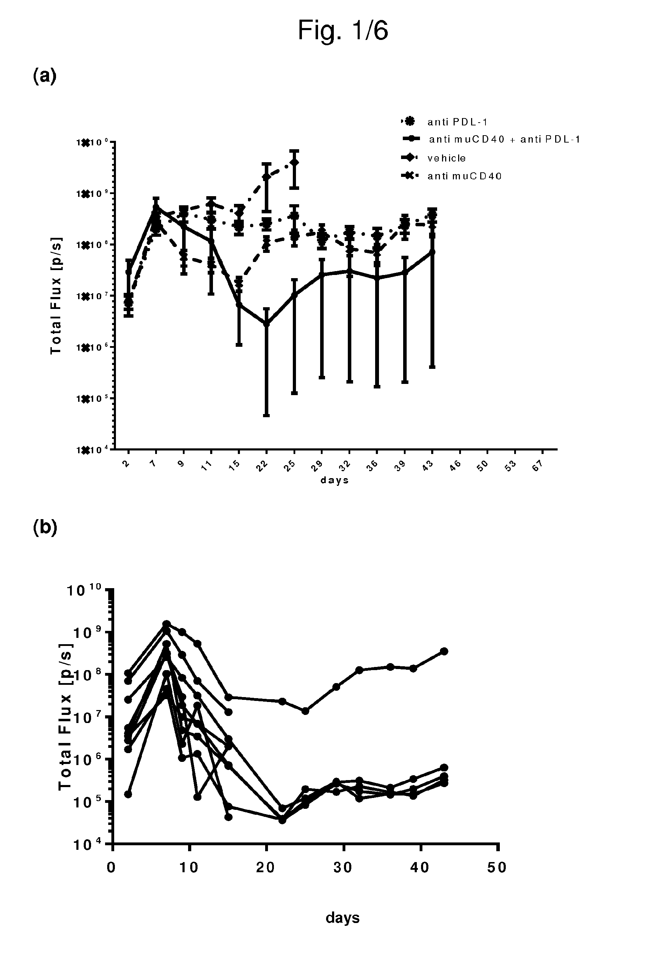

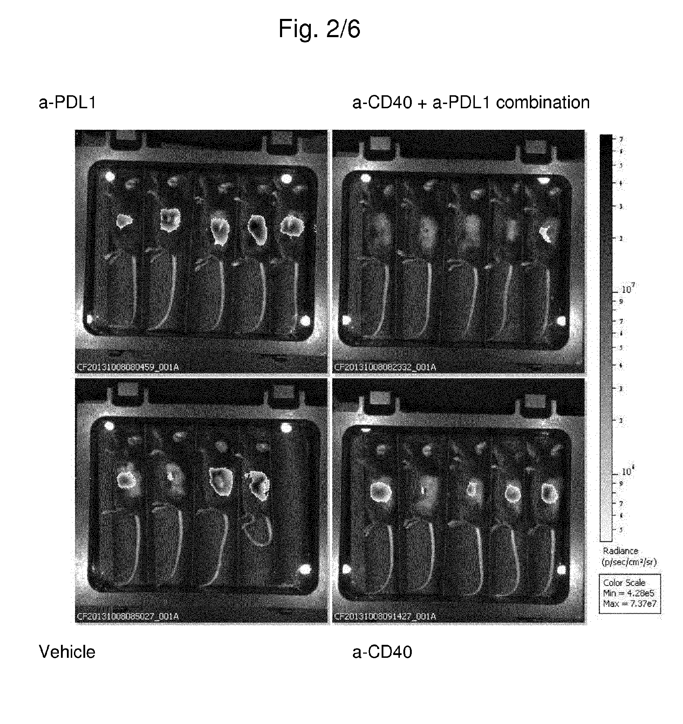

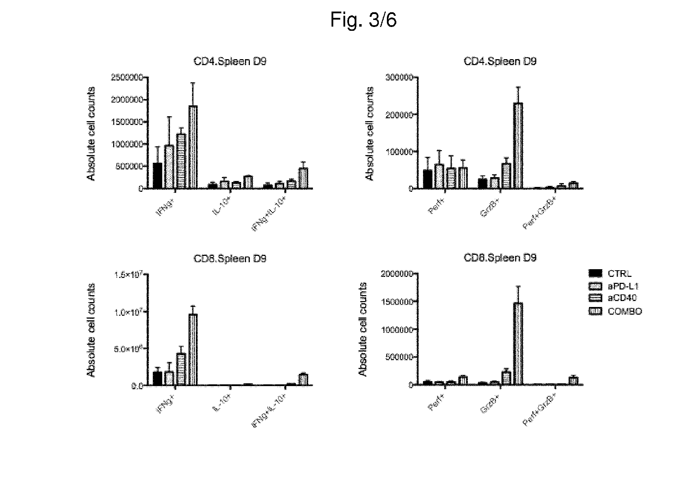

The combination of muFGK4.5 with anti-PD-L1 antibody exhibited marked tumor growth inhibition compared to vehicle and anti-PD-L1 in the syngeneic orthotopic Panc02 model. Moreover, muFGK4.5 and anti-PD-L1 antibody together synergistically enhanced the expansion of effector T-cells in vivo, translating into complete elimination of tumors in 4 out of 9 mice in this model (**Fig. 1** **and** **2**). Immune Pharmacodynamic (PD) analysis demonstrated that muFGK4.5 increased the number of activated T cells (CD4 and CD8) approximately 2 fold in mice spleen and blood , as well as in lymph nodes. Interestingly, the combination of muFGK4.5 with anti PD L1 antibody further enhanced the number of activated T cells in mouse spleen approximately 6 to 7 fold after 9 days of initiating therapy (**Fig. 3**).

### Example 2

This Example demonstrates the efficacy of the combination of a-PDL-1 and a-CD40 with two different anti-cytokines: a-IL6 and a-TNFa alone or together. This information is useful as those antibodies may be used to neutralize cytokine release after a-CD40 injection.

### Materials and Methods

### Cell culture and application

Panc-02-Fluc cells clone H7 (human pancreatic carcinoma cells) were originally obtained from ATCC (American Type Culture Collection) and after expansion deposited in an internal cell bank. Panc-02-Fluc cells were cultured in RPMI medium containing 10% FCS (Sigma) + 1% glutamax + 500ug/ml hygromicin. The cells were cultured at 37°C in a water-saturated atmosphere at 5 % CO₂.

A small incision was made in the left flank of the abdomen of anesthetized C57BL/6 mice. The peritoneal wall was opened and the pancreas carefully isolated with forceps. Ten microliters (0.2x106 cells in RPMI medium) of cell suspension were injected into the tail of the pancreas. Peritoneal wall and skin wounds were closed using 5/0 resolvable suture.

### Animals

Sixty C57BL/6 female mice; age 8-9 weeks at start of experiment (purchased from Charles Rivers, Germany) were maintained under specific-pathogen-free condition with daily cycles of 12 h light /12 h darkness according to committed guidelines (GV-Solas; Felasa; TierschG). Experimental study protocol was reviewed and approved by local government (P 2011-128). After arrival, animals were maintained for one week to get accustomed to new environment and for observation. They were afterwards implanted with a transponder subcutaneously on the right side of the back for identification and maintained one more week for recovery. Continuous health monitoring was carried out on regular basis.

### Treatment

Mice were injected i.panc. on study day 0 with 2x10⁵ of PancO2-Fluc cells in RPMI (passage 16 at a viability of 90.5%).

At day 6, 7, 8, and 9, 12 mice/group were injected i.p. with the different compounds. At days 7, 14 and 21, 10 mice/group were injected i.p. with the different compounds (see study groups).

### Test compounds (antibodies)

### Specification of compounds

| Compound | Conc. (mg/mL) | Formulation buffer |
|---|---|---|
| muIgG1 CD40 FGK4.5 B6 (Component A) | 3.62 | 20 mM Histidine, 140 mM NaCl, 0.01% Tween20, pH6.0 |
| PD-L1 6E11 muIgG1 (Component B) | 27.1 | 20 mM Histidine Acetate, 240 mM Sucrose, 0.02% Tween20, pH 5.5 |
| PD-L1 6E11 muIgG1 (Component B) | 14.9 | 20 mM Histidine Acetate, 240 mM Sucrose, 0.02% Tween20, pH 5.5 |
| MP5-20F3 (a-IL6) (Component C) | 5.08 | PBS, pH 7 |
| TN3-19.12 (a-TNFa) (Component C) | 4.62 | PBS, pH 7 |

All mice were injected with 200 µL of the appropriate solution. All antibodies were injected i.p. The mice in the vehicle group were injected with Histidine buffer and the treatment group with the antibody. To obtain the proper amount of antibody per 200 µL, the antibody solutions were diluted with Histidine buffer when necessary. The maximum number of treatments for a mouse was four times.

### Investigations

### Monitoring

Animals were controlled daily for clinical symptoms and detection of adverse effects. Termination criteria for animals were clinical sickness, impaired locomotion, scruffy fur.

### Serum extraction for compound analysis

Serum samples from half treated animals were collected 1h after the 2^{nd}, 5^{th} and 6^{th} antibodies treatments and the other half 24h after the 2^{nd}, 5^{th} and 6^{th} antibodies treatments. All animal were bled at day of sacrifice. Samples were stored at -20° C.

### Autopsy

Mice were sacrificed according to the termination criteria. From four mice/group spleen, liver and pancreas tumors were harvested for subsequent histopathological analysis (4% formaldheyde, snap frozen).

### Sample processing

The pancreas tumors, liver and spleen were resected and fixed immediately in 10 % formalin solution and subsequently processed for paraffin embedding (Leica Automatic Tissue Processor TP1020, Germany) and microtone 4µm sectioning (Leica RM2235 Rotary Microtome, Germany). Haematoxylin and Eosin staining was performed using standard protocol. Murine immune cells were detected using rat anti-mouse CD68 (AbD Serotec, Switzerland), in accordance with the manufacturer's instructions.

### Biochemical Analysis

### mCD40 IgG-ELISA and mPDL-1 IgG ELISA

Anti-CD40 concentrations in serum were tested using a CD40/Fc Chimera (R&D, 1215-CD-050) ELISA for quantification of anti-CD40, according to the protocol (Roche-Glycart, Switzerland). The reagents; Capture Protein: 6xHis tag Biotin (Abcam, ab27025) and CD40/Fc Chimera (R&D, 1215-CD-050), Detection Antibody: Anti-mouse IgG (HRP), (Abcam, ab98808) were used. Serum samples were analyzed in serial dilutions (1:300, 1:1500, 1:7500). Absorption was measured using a measuring wavelength of 405 nm and a reference wavelength of 490 nm (VersaMax tunable microplate reader, Molecular Devices).

Anti-PD-L1 concentrations in serum were tested using a murine PD-L1 (R&D, 1019-B7-100) ELISA for quantification of anti-PD-L1, according to the protocol (Roche-Glycart, Switzerland). The reagents; Capture Protein: IgG-FC Biotin (Abcam ab98561) and murine B7-H1/PD-L1 (R&D, 1019-B7-100), Detection Antibody: Anti-mouse IgG (HRP), (Abcam, ab98808) were used. Serum samples were analyzed in serial dilutions (1:2500, 1:12500, 1:62500). Absorption was measured using a measuring wavelength of 405 nm and a reference wavelength of 490 nm (VersaMax tunable microplate reader, Molecular Devices).

### Cytokine Analysis

Cytokines were measured by Luminex assay (Bio-Plex Pro mouse cyokine 23-plex assay), according to manufacturer's instruction.

### Results

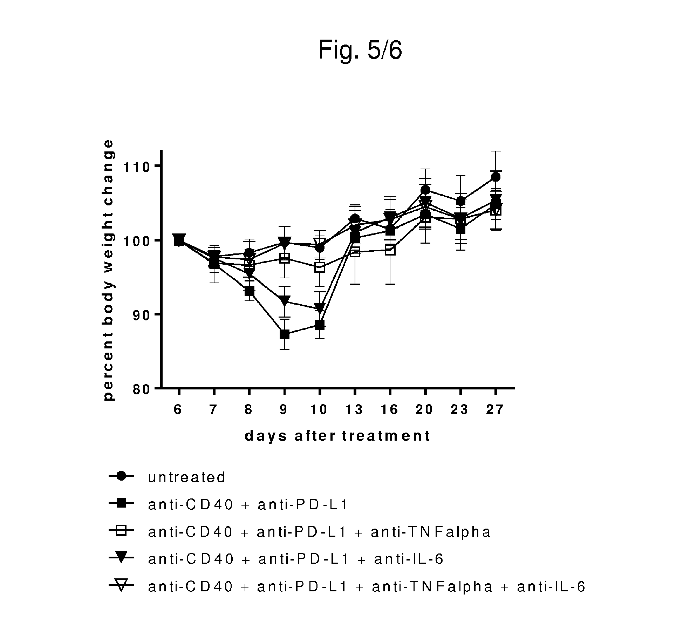

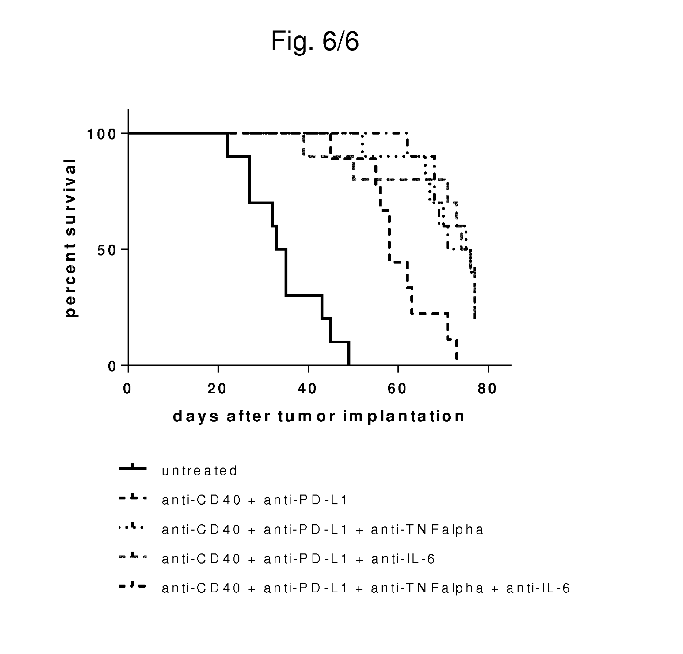

The addition of anti-cytokines as a third component results in an improved tolerability, demonstrated by less body weight loss compared to the doublet of CD40 agonist and PD-L1 inhibitor alone (Components A and B, respectively). In particular the addition of anti-TNF alpha as the third component (Component C) results in improved tolerability, as for example demonstrated by monitoring of body weight loss during treatment (see **Fig. 5**). At the same time, the activity of the CD40/PD-L1 combination is preserved when injected together with an anti-cytokine as third component (see **Fig. 6**).

## Claims

1. A pharmaceutical product comprising A) a first component comprising as an active ingredient an antibody or an antigen-binding portion thereof that specifically binds to and activates human CD40; and B) a second component comprising as an active ingredient a PD-L1 antibody; for the combined, sequential or simultaneous, treatment of a proliferative disease, in particular cancer

2. The pharmaceutical product according to claim 1, comprising
A) a first component comprising as an active ingredient an anti-CD40 antibody comprising a VL of SEQ ID NO:3 and a VH of SEQ ID NO:4; or
an antibody comprising the heavy chain - and light chain variable domain amino acid sequences of antibody 21.4.1 (ATCC Deposit No. PTA-3605); and
B) a second component comprising as an active ingredient a PD-L1 antibody selected from an antibody comprising
a) a heavy chain variable domain VH of SEQ ID NO:5 and a light chain variable domain VL of SEQ ID NO:8, or
b) a heavy chain variable domain VH of SEQ ID NO:6 and a light chain variable domain VL of SEQ ID NO:9, or
c) a heavy chain variable domain VH of SEQ ID NO:6 and a light chain variable domain VL of SEQ ID NO:10, or
d) a heavy chain variable domain VH of SEQ ID NO:6 and a light chain variable domain VL of SEQ ID NO: 11, or
e) a heavy chain variable domain VH of SEQ ID NO:6 and a light chain variable domain VL of SEQ ID NO: 12, or
f) a heavy chain variable domain VH of SEQ ID NO:6 and a light chain variable domain VL of SEQ ID NO: 13, or
g) a heavy chain variable domain VH of SEQ ID NO:6 and a light chain variable domain VL of SEQ ID NO:14, or
h) a heavy chain variable domain VH of SEQ ID NO:6 and a light chain variable domain VL of SEQ ID NO: 15, or
i) a heavy chain variable domain VH of SEQ ID NO:6 and a light chain variable domain VL of SEQ ID NO:16, or
j) a heavy chain variable domain VH of SEQ ID NO:6 and a light chain variable domain VL of SEQ ID NO: 17, or
k) a heavy chain variable domain VH of SEQ ID NO:6 and a light chain variable domain VL of SEQ ID NO:18, or
l) a heavy chain variable domain VH of SEQ ID NO:6 and a light chain variable domain VL of SEQ ID NO:19, or
m) a heavy chain variable domain VH of SEQ ID NO:6 and a light chain variable domain VL of SEQ ID NO:20, or
n) a heavy chain variable domain VH of SEQ ID NO:6 and a light chain variable domain VL of SEQ ID NO:21, or
o) a heavy chain variable domain VH of SEQ ID NO:6 and a light chain variable domain VL of SEQ ID NO:22, or
p) a heavy chain variable domain VH of SEQ ID NO:7 and a light chain variable domain VL of SEQ ID NO:23.

3. The pharmaceutical product according to claim 1 or 2, comprising A) a first component comprising as an active ingredient an antibody comprising a VL of SEQ ID NO:3 and a VH of SEQ ID NO:4; and B) a second component comprising as an active ingredient an antibody comprising a VL of SEQ ID NO:8 and a VH of SEQ ID NO:5; for the combined, sequential or simultaneous, treatment of a proliferative disease, in particular cancer.

4. The pharmaceutical product according to claim 1 to 3, wherein components A) and B) are administered separately.

5. The pharmaceutical product according to claim 4, wherein component A and/or B is administered intravenously (i.v.) or subcutaneously (s.c.).

6. The pharmaceutical product according to claim 5, wherein component A is administered at fixed doses between 4 and 16mg, and component B is administered at a fixed dose of 1200mg.

7. The pharmaceutical product according to claim 6, wherein administration of components A and B is separated by 1 to 42 days, preferably by 1, or 7, or 14 or 21 or 42 days.

8. The pharmaceutical product according to claim 7, wherein component A is administered from 1 to 4 times together with component B, and subsequently treatment continues only with component B until disease progression.

9. The pharmaceutical product according to any one of claims 1 to 8 for the treatment of cancer, preferably solid tumors.

10. The use of an antibody, or an antigen-binding portion thereof, that specifically binds to and activates human CD40; and a PD-L1 antibody for the manufacture of a medicament for the combined, sequential or simultaneous, treatment of a proliferative disease, such as cancer preferably solid tumors.

11. The pharmaceutical product according to claim 1, further comprising as a third component (C) a cytokine inhibitor.

12. The pharmaceutical product according to claim 11, wherein the cytokine inhibitor is an anti-TNF alpha antibody.

13. A kit comprising a pharmaceutical product according to any one of claims 1 to 12 together with instructions how to use it.

14. A method for treating a patient having cancer comprising administering to said patient a pharmaceutical product according to any one of claims 1 to 12.

15. The novel products, methods and uses substantially as described herein.
